# EUROPEAN PATENT APPLICATION

(11) **EP 4 670 617 A1**
(43) Date of publication of application: **31.12.2025**
(21) Application number: 23930838.0
(22) Date of filing: 17.11.2023
(51) Int. Cl.: A61B 3/15

(54) **OPHTHALMIC APPARATUS AND OPERATING METHOD OF OPHTHALMIC APPARATUS**

(30) Priority: 28.03.2023 JP 2023052056
(71) Applicant: Topcon Corporation, Tokyo 174-8580 (JP)
(72) Inventor: MOCHIZUKI, Yuki, Tokyo 174-8580 (JP); SHIBANO, Takaaki, Tokyo 174-8580 (JP); SUZUKI, Minami, Tokyo 174-8580 (JP); YAMABE, Masaru, Tokyo 174-8580 (JP); TSUKIHARA, Kouichi, Tokyo 174-8580 (JP)
(74) Representative: Schmitt-Nilson Schraud Waibel Wohlfrom Patentanwälte Partnerschaft mbB
(86) International application number: PCT/JP2023/041451
(87) International publication number: WO 2024/202211

(57) **Abstract**

There are provided an ophthalmic device capable of examining on a subject eye by an examination head without bringing the examination head closer to a nose of a subject and a method for operating the ophthalmic device. The ophthalmic device includes a displacement mechanism (an XZ movement mechanism (16), a Y movement mechanism (18), a swing rotation mechanism (20), and a tilt rotation mechanism (80)) configured to displace an examination head (66) with respect to a subject eye (E), a nose photographing controlling unit (41) configured to cause at least two cameras (34a) to photograph a nose of a subject from a plurality of directions different from each other, a nose position detecting unit (42) configured to detect a position of the nose (N) based on respective photographed images of the nose (N) from the cameras (34a), a tilting angle determining unit (43) configured to determine a tilting angle (θ) of an axis (TA) of tilt with respect to a reference axis (VA) based on a result of detection by the nose position detecting unit (42), and a drive controlling unit (46) configured to drive the displacement mechanism to displace the examination head (22) to an examination position for the subject eye (E) along the axis (TA) of tilt with the tilting angle (θ) determined by the tilting angle determining unit (43).

## Description

### {Technical Field}

The presently disclosed subject matter relates to an ophthalmic device which aligns an examination head with a subject eye and a method for operating the ophthalmic device.

### {Background Art}

In ophthalmology, ophthalmic examinations (e.g., acquisition of various ocular characteristics, such as ocular refractive power, an intraocular pressure, and the number of corneal endothelial cells, of a subject eye, fundus imaging, and tomography) on the subject eye are performed by an ophthalmic device. Alignment of an examination head (also referred to as an examination unit) of the ophthalmic device with respect to the subject eye is extremely important in terms of, e.g., accuracy, reliability, and image quality of a result of the examinations on the subject eye.

For this reason, each of the ophthalmic devices according to Patent Literatures 1 and 2 stereoscopically photographs a subject eye using a stereo camera and executes alignment detection that detects a relative position of the subject eye to an examination head based on anterior eye part images of the subject eye obtained by the stereoscopic photographing. The ophthalmic device moves the examination head through electromotive driving based on a result of the alignment detection, thereby executing automatic alignment of the examination head with the subject eye.

### {Citation List}

### {Patent Literature}

{Patent Literature 1} Japanese Patent Application Laid-Open No. 2013-248376
{Patent Literature 2} Japanese Patent Application Laid-Open No. 2021-069415

### {Summary of Invention}

### {Technical Problem}

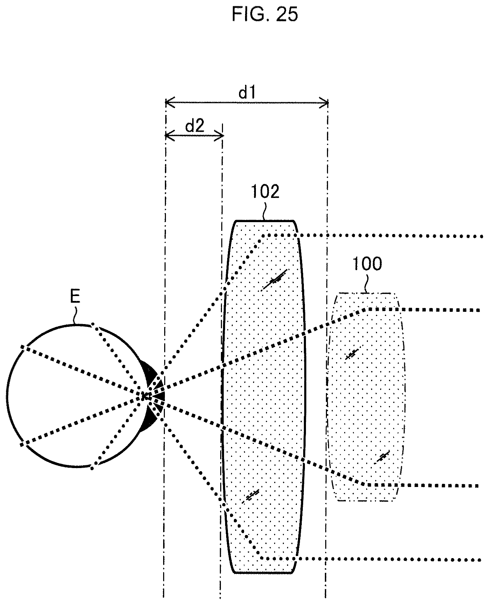

Figure 25 is an explanatory diagram for explaining a relationship between a lens size of an objective lens in an examination head and an operating distance of the examination head. As illustrated in Figure 25, a photographic angle of view of a conventional objective lens 100 in an examination head is about 45°. An ophthalmic device such as a fundus camera, an Optical Coherence Tomography (OCT) device, or a Scanning Laser Ophthalmoscope (SLO) device supporting wide-angle photographing, has an objective lens 102 larger than the objective lens 100 in an examination head due to optical constraints. As a result, an operating distance d2 between a subject eye E and the examination head with the objective lens 102 is shorter than an operating distance d1 with the objective lens 100.

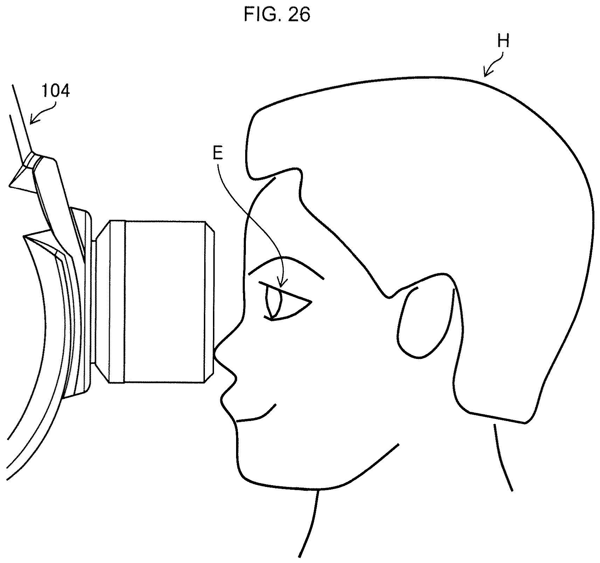

Figure 26 is an explanatory view for explaining a problem arising from a short operating distance between the subject eye E and an examination head 104. For example, when an objective lens diameter of the examination head 104 is 80 mm, and an operating distance of the examination head 104 is 50 mm, as illustrated in Figure 26, a distance between a forehead of the subject H and the examination head 104 is about 22 mm, a distance between a nose of the subject H and the examination head 104 is about 2 mm, and a distance between cheeks of the subject H and the examination head 104 is about 28 mm. The distance between the nose of the subject H and the examination head 104 is thus particularly short. As a result, in executing automatic alignment of the examination head 104 with the subject eye E using the methods according to Patent Literatures 1 and 2 described above, the examination head 104 needs to be brought closer to the nose of the subject H.

The presently disclosed subject matter has been made in view of the above-described circumstances, and has as its object to provide an ophthalmic device capable of examining on a subject eye through an examination head without bringing the examination head closer to a nose of a subject and a method for operating the ophthalmic device.

### {Solution to Problem}

An ophthalmic device for achieving the object of the presently disclosed subject matter includes: an examination head configured to examine a subject eye; a displacement mechanism configured to displace the examination head with respect to the subject eye; a plurality of cameras provided at the examination head; a nose photographing controlling unit configured to cause at least two of the cameras to photograph a nose of a subject from a plurality of directions different from each other; a nose position detecting unit configured to detect a relative position of the nose to the examination head based on respective photographed images of the nose photographed by the cameras; a tilting angle determining unit configured to, when an axis along an eye direction of the subject eye parallel to a front-back direction which is an operating distance direction of the examination head is a reference axis, and an axis obtained by tilting the reference axis in an outward direction away from the nose of the subject around the subject eye is an axis of tilt, determine a tilting angle of the axis of tilt with respect to the reference axis based on a result of detection by the nose position detecting unit; and a drive controlling unit configured to drive the displacement mechanism to displace the examination head to an examination position for the subject eye along the axis of tilt with the tilting angle determined by the tilting angle determining unit.

According to the ophthalmic device, it is possible to displace the examination head to the examination position for the subject eye without bringing the examination head closer to the nose of the subject.

In the ophthalmic device according to another aspect of the presently disclosed subject matter, the nose photographing controlling unit causes all of the cameras to simultaneously photograph the nose after positionally adjusting, by the displacement mechanism, the examination head to a position where the nose is photographable by all the cameras. This makes it possible to precisely execute detection of the relative position of the nose by the nose position detecting unit.

In the ophthalmic device according to another aspect of the presently disclosed subject matter, the nose photographing controlling unit executes a divisional photographing process of causing one or more of the cameras to photograph the nose after driving the displacement mechanism to move the examination head to a position where the nose is photographable by the one or more cameras, a position information acquiring process of acquiring photographing position information of each of the cameras that have photographed the nose in the divisional photographing process and a repetition process of repeatedly executing the divisional photographing process and the position information acquiring process until photographing of the nose by all the cameras is completed, and the nose position detecting unit detects the relative position of the nose based on the respective photographed images from and the respective pieces of photographing position information of the cameras. This makes it possible to precisely execute detection of the relative position of the nose by the nose position detecting unit even if the nose is not simultaneously photographable by all the cameras.

In the ophthalmic device according to another aspect of the presently disclosed subject matter, the tilting angle determining unit computes, for each of a plurality of tilting angles different from each other and constituting the tilting angle, a nose distance which is a distance between the examination head and the nose when the ophthalmic device brings the examination head closer to an operating distance determined in advance from the subject eye based on a result of detection by the nose position detecting unit and determines the tilting angle based on a result of computation of the respective nose distances for the tilting angles. This makes it possible to determine a tilting angle which allows displacement of the examination head to the examination position for the subject eye without bringing the examination head closer to the nose of the subject.

In the ophthalmic device according to another aspect of the presently disclosed subject matter, the plurality of cameras photograph an anterior eye part of the subject eye halfway through displacement of the examination head to the examination position by the displacement mechanism, the device includes an alignment detection unit configured to detect a relative position of the subject eye to the examination head based on anterior eye part images of the subject eye photographed by the plurality of cameras, and the drive controlling unit drives the displacement mechanism based on a result of detection by the alignment detection unit to execute alignment of the examination head with the subject eye while maintaining the tilting angle determined by the tilting angle determining unit. This makes it possible to displace the examination head to the examination position for the subject eye without bringing the examination head closer to the nose of the subject.

In the ophthalmic device according to another aspect of the presently disclosed subject matter, the examination head includes an objective lens and a lens-barrel configured to hold the objective lens, and one of the plurality of cameras is provided at a position below the objective lens in a distal end face of the lens-barrel. This makes it possible to prevent each camera from coming closer to the nose of the subject.

In the ophthalmic device according to another aspect of the presently disclosed subject matter, the cameras are provided at the position below the objective lens and positions on the left and right of the objective lens in the distal end face of the lens-barrel. This makes it possible to prevent each camera from coming closer to the nose of the subject and a pupil of the subject eye from being hidden by upper lashes of the subject at the time of photographing of the subject eye by each camera.

In the ophthalmic device according to another aspect of the presently disclosed subject matter, the displacement mechanism includes a movement mechanism configured to move the examination head with respect to the subject eye in the front-back direction, a left-right direction, and an up-down direction, and a rotation mechanism configured to rotate the examination head around a rotation axis determined in advance. This makes it possible to arbitrarily displace the examination head with respect to the subject eye.

In the ophthalmic device according to another aspect of the presently disclosed subject matter, the rotation axis is parallel to the up-down direction, and the outward direction is parallel to the left-right direction.

In the ophthalmic device according to another aspect of the presently disclosed subject matter, the rotation axis is perpendicular to the up-down direction, and the outward direction is an upward direction of the up-down direction.

A method for operating an ophthalmic device for achieving the object of the presently disclosed subject matter is a method for operating an ophthalmic device including an examination head configured to examine a subject eye, a displacement mechanism configured to displace the examination head with respect to the subject eye, and a plurality of cameras provided to be movable integrally with the examination head, the method including a nose photographing controlling step of causing at least two of the cameras to photograph a nose of a subject from a plurality of directions different from each other, a nose position detecting step of detecting a relative position of the nose to the examination head based on respective photographed images of the nose photographed by the cameras, a tilting angle determining step of, when an axis along an eye direction of the subject eye parallel to a front-back direction which is an operating distance direction of the examination head is a reference axis, and an axis obtained by tilting the reference axis in an outward direction away from the nose of the subject around the subject eye is an axis of tilt, determining a tilting angle of the axis of tilt with respect to the reference axis based on a result of detection in the nose position detecting step, and a drive controlling step of driving the displacement mechanism to displace the examination head to an examination position for the subject eye along the axis of tilt with the tilting angle determined in the tilting angle determining step.

### {Advantageous Effects of Invention}

According to the presently disclosed subject matter, it is possible to examine a subject eye by an examination head without bringing the examination head closer to a nose of a subject.

### {Brief Description of Drawings}

Figure 1 is a side view of an ophthalmic device according to a first embodiment;
Figure 2 is a front view of a lens-barrel as viewed from a front side in a Z direction;
Figure 3 is a cross-sectional view of the lens-barrel taken along line 3-3 in Figure 2;
Figure 4 is a block diagram illustrating a configuration of the ophthalmic device according to the first embodiment;
Figure 5 is an explanatory diagram for explaining automatic alignment of an examination head;
Figure 6 is an explanatory diagram for explaining nose photographing control using a stereo camera by a nose photographing controlling unit and is a diagram of an objective lens and the stereo camera as viewed from an upper side in a Y direction;
Figure 7 is an explanatory view for explaining photographed nose images photographed by three cameras;
Figure 8 is an explanatory diagram for explaining a case where a nose is not simultaneously photographable by three cameras and is a diagram of the objective lens and the stereo camera as viewed from the upper side in the Y direction;
Figure 9 is an explanatory diagram for explaining an example of a method for determining a tilting angle by a tilting angle determining unit;
Figure 10 is an explanatory diagram for explaining an example 1-1 of the automatic alignment of the examination head according to the first embodiment;
Figure 11 is an explanatory diagram for explaining an example 1-2 of the automatic alignment of the examination head according to the first embodiment;
Figure 12 is an explanatory diagram for explaining an example 1-3 of the automatic alignment of the examination head according to the first embodiment;
Figure 13 is a flowchart illustrating a flow of a process of examining a subject eye by the ophthalmic device according to the first embodiment;
Figure 14 is a flowchart illustrating a flow of a process of determining the tilting angle;
Figure 15 is a flowchart illustrating a flow of an automatic alignment process for the examination head;
Figure 16 is an explanatory diagram for explaining displacement of the examination head after the start of the automatic alignment;
Figure 17 is a side view of an ophthalmic device according to a second embodiment;
Figure 18 is an explanatory diagram for explaining an example 2-1 of automatic alignment of an examination head according to the second embodiment;
Figure 19 is an explanatory diagram for explaining an example 2-2 of the automatic alignment of the examination head according to the second embodiment;
Figure 20 is an explanatory diagram for explaining another method for determining the tilting angle by the tilting angle determining unit;
Figure 21 is a side view of an ophthalmic device according to a third embodiment;
Figure 22 is an explanatory diagram for explaining an example 3 of automatic alignment of an examination head according to the third embodiment;
Figure 23 is a side view of an ophthalmic device according to a fourth embodiment;
Figure 24 is an explanatory diagram for explaining an example 4 of automatic alignment of an examination head according to the fourth embodiment;
Figure 25 is an explanatory diagram for explaining a relationship between a lens size of an objective lens in an examination head and an operating distance of the examination head; and
Figure 26 is an explanatory view for explaining a problem arising from a short operating distance between a subject eye and an examination head.

### {Description of Embodiments}

### [First Embodiment]

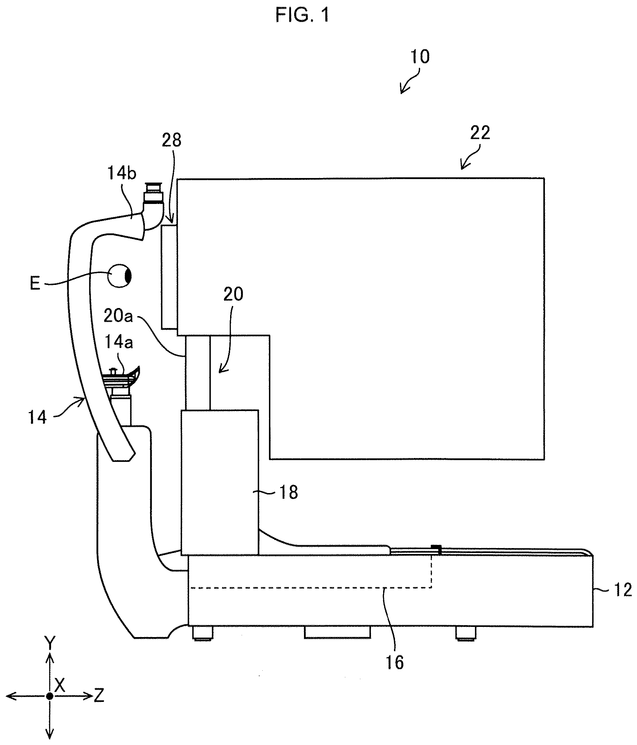

Figure 1 is a side view of an ophthalmic device 10 according to a first embodiment. An X direction in Figure 1 is a left-right direction based on a subject, a Y direction is an up-down direction, and that a Z direction is a front-back direction (also referred to as an operating distance direction) parallel to a forward direction toward the subject (a subject eye E) and a rearward direction away from the subject.

As illustrated in Figure 1, the ophthalmic device 10 is a multifunction machine which is a combination of a fundus camera that executes fundus imaging of the subject eye E and an optical coherence tomograph that obtains a tomographic image of the subject eye E using OCT. The ophthalmic device 10 includes a base 12, a face support 14, an XZ movement mechanism 16, a Y movement mechanism 18, a swing rotation mechanism 20, and an examination head 22.

The face support 14 is attached to a front end portion on a front side (a subject eye E side) in the Z direction of the base 12. The XZ movement mechanism 16 is also provided at the base 12.

The face support 14 includes a chin rest 14a and a forehead rest 14b which are positionally adjustable in the Y direction (up-down direction), and supports a face of the subject at a position facing the examination head 22 (a lens-barrel 28).

The XZ movement mechanism 16 together with the Y movement mechanism 18 (to be described later) constitutes a movement mechanism according to the presently disclosed subject matter. The XZ movement mechanism 16 includes a pedestal which is movable in each of the X and Z directions with respect to the base 12 and an electric drive mechanism (a publicly known actuator, such as a motor drive mechanism) which moves the pedestal in each of the X and Z directions (both not illustrated). The XZ movement mechanism 16 integrally moves the Y movement mechanism 18, the swing rotation mechanism 20, and the examination head 22 in the X and Z directions.

The Y movement mechanism 18 includes a lifting table which is movable in the Y direction and an electric drive mechanism which moves the lifting table in the Y direction (both not illustrated). The Y movement mechanism 18 integrally moves the swing rotation mechanism 20 and the examination head 22 in the Y direction. With this configuration, the XZ movement mechanism 16 and the Y movement mechanism 18 can integrally move the swing rotation mechanism 20 and the examination head 22 in the X, Y, and Z directions.

The swing rotation mechanism 20 corresponds to a rotation mechanism according to the presently disclosed subject matter and, together with the XZ movement mechanism 16 and the Y movement mechanism 18 described earlier, constitutes a displacement mechanism according to the presently disclosed subject matter. The swing rotation mechanism 20 includes a rotating shaft 20a parallel to the Y direction and an electric drive mechanism which rotates the rotating shaft 20a, and rotates (swings) the examination head 22 around the rotating shaft 20a.

The examination head 22 is attached to an upper end portion in the Y direction of the rotating shaft 20a. With this configuration, the examination head 22 is movable in the X, Y, and Z direction by the XZ movement mechanism 16 and the Y movement mechanism 18, and is rotatable in a direction around the rotating shaft 20a by the swing rotation mechanism 20.

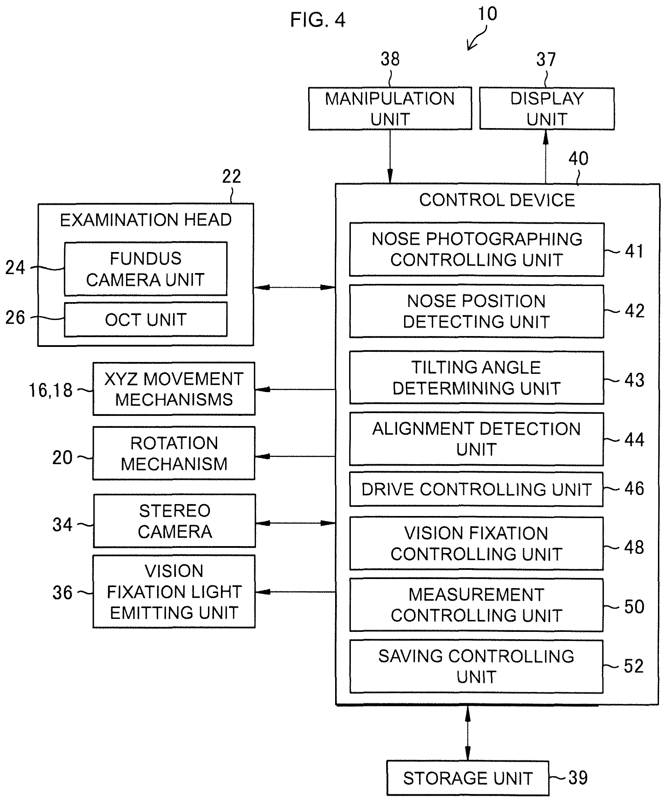

The examination head 22 includes a fundus camera unit 24 and an OCT unit 26 (to be described later) illustrated in Figure 4, and the lens-barrel 28. The fundus camera unit 24 photographs a fundus of the subject eye E through an objective lens 30 (to be described later) and outputs a fundus image which is a frontal image of the fundus to a control device 40 (to be described later) (see Figure 4). The OCT unit 26 performs OCT imaging of the subject eye E through the objective lens 30 and outputs a signal such as a detection signal needed to generate a tomographic image of the subject eye E to the control device 40. Specific configurations of the fundus camera unit 24 and the OCT unit 26 are publicly known techniques (see Patent Literature 1 described above) and that a specific description thereof will be omitted.

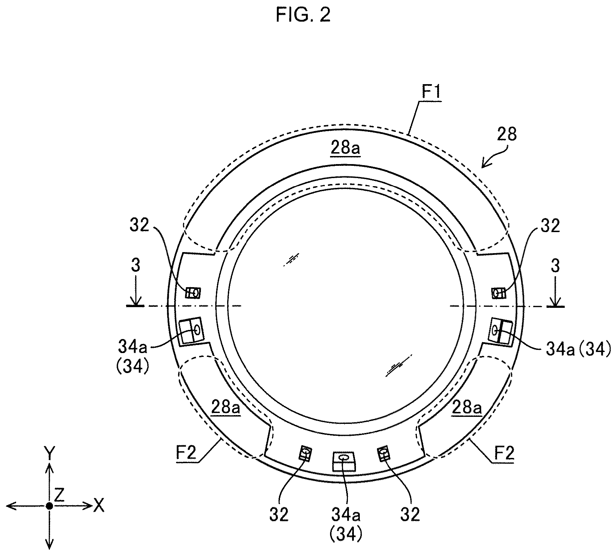

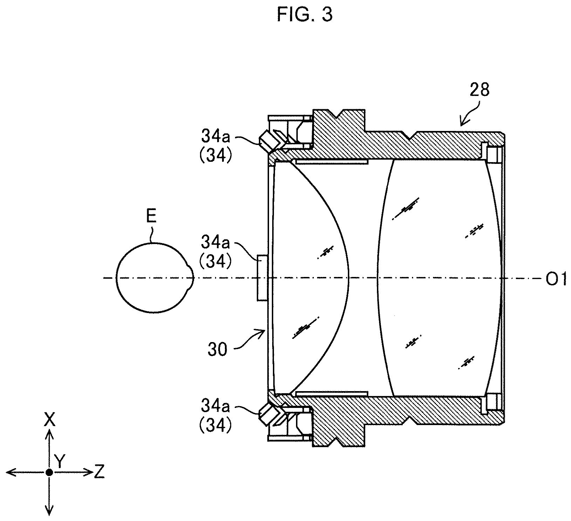

Figure 2 is a front view of the lens-barrel 28 as viewed from the front side in the Z direction. Figure 3 is a cross-sectional view of the lens-barrel 28 taken along line 3-3 in Figure 2. Figures 1 to 3 illustrates an example which provides the lens-barrel 28 at an end portion on the front side in the Z direction of the examination head 22. The lens-barrel 28 houses (holds) the objective lens 30 having an optical axis O1 (see Figure 3) parallel to the Z direction. Four illumination light sources 32 and a stereo camera 34 are provided at a lens-barrel distal end face 28a on the front side in the Z direction of the lens-barrel 28. As the objective lens 30, for example, a large lens supporting wide-angle photographing, i.e., a lens with a short operating distance is used (see Figure 25). The type of the objective lens 30 is not particularly limited and that a lens with a photographic angle of view of about 45° may be used.

When the objective lens 30 and the rotating shaft 20a described earlier are viewed from a one-direction side in the Y direction, a position of the rotating shaft 20a and a position of the objective lens 30 coincide (the term "coincide" as used herein is intended to include the meaning of "substantially coincide"; the same applies hereinafter). With this configuration, the examination head 22 is rotated (swung) around the objective lens 30 by the swing rotation mechanism 20.

Respective illumination light sources 32 are provided at two end portions in the X direction (two left and right end portions) of the lens-barrel distal end face 28a, and two illumination light sources 32 are provided at a lower end portion of the lens-barrel distal end face 28a such that a camera 34a (to be described later) is sandwiched therebetween. Each illumination light source 32 is, for example, a Light Emitting Diode (LED) light source and illuminates the subject eye E.

The stereo camera 34 is used for alignment detection that detects relative positions in the X, Y, and Z directions of the subject eye E to the examination head 22. The stereo camera 34 includes a plurality of cameras 34a. For example, in the present embodiment, the stereo camera 34 includes two cameras 34a provided at the two end portions in the X direction (the two left and right end portions) corresponding to positions on the left and right of the objective lens 30 in the lens-barrel distal end face 28a and one camera 34a provided at the lower end portion corresponding to a position below the objective lens 30 in the lens-barrel distal end face 28a, three cameras 34a in total. The cameras 34a simultaneously photograph an anterior eye part of the subject eye E from a plurality of (three in the present embodiment) directions different from each other at the time of the alignment detection and output a plurality of (three) anterior eye part images of the subject eye E to the control device 40 (see Figure 4). The number of cameras 34a may be two, or four or more.

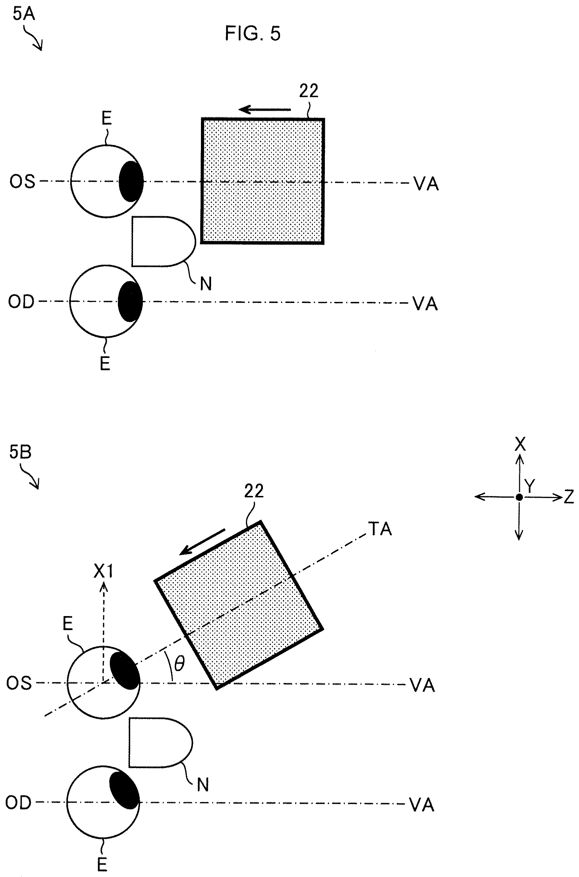

Positions of the cameras 34a may be appropriately changed. For example, if the camera 34a is provided in an upper region F1 (see Figure 2) above (in the Y direction) the two end portions in the X direction in the lens-barrel distal end face 28a, a pupil of the subject eye E may be hidden by upper lashes of the subject in photographing the subject eye E by the camera 34a. Additionally, if respective cameras 34a are provided in left and right lower oblique regions F2 (see Figure 2) between the two end portions in the X direction and the lower end portion in the lens-barrel distal end face 28a, each camera 34a is likely to come closer to a nose N (see Figure 5) of the subject during alignment of the examination head 22. For this reason, the cameras 34a are preferably provided at the two end portions in the X direction and the lower end portion of the lens-barrel distal end face 28a.

Figure 4 is a block diagram illustrating a configuration of the ophthalmic device 10 according to the first embodiment. As illustrated in Figure 4, the ophthalmic device 10 includes a vision fixation light emitting unit 36, a display unit 37, a manipulation unit 38, a storage unit 39, and the control device 40 in addition to the XZ movement mechanism 16, the Y movement mechanism 18, the swing rotation mechanism 20, the examination head 22, and the stereo camera 34 described earlier.

The vision fixation light emitting unit 36 guides and fixes an eye direction of the subject eye E by emitting vision fixation light (a bright spot image) toward the subject eye E. The vision fixation light emitting unit 36 includes a publicly known vision fixation target display unit, a plurality of vision fixation holes, and an external fixation lamp (all not illustrated) (see Patent Literature 2 described above).

The vision fixation target display unit is provided inside the examination head 22 and is used for internal vision fixation that projects vision fixation light (e.g., a bright spot image) onto the subject eye E through the objective lens 30. The vision fixation holes are provided at a front surface in the Z direction (which may be the lens-barrel distal end face 28a) of the examination head 22 so as to surround the objective lens 30 and are used for peripheral vision fixation. The peripheral vision fixation is a vision fixation method for selectively lighting the vision fixation holes, thereby causing the subject eye E to make a great circumnutation in a desired direction. The external fixation lamp is provided at the face support 14 or the examination head 22 and is used for external vision fixation. The external vision fixation is a vision fixation method for causing the subject eye E to make a circumnutation in an arbitrary direction or make a greater circumnutation than under internal vision fixation by adjusting a light source position of the external fixation lamp, or adjusting an orientation of the subject eye E by guiding a visual line of the subject eye E or a fellow eye when the internal vision fixation cannot be performed.

As the display unit 37, which is a type of display device, for example, a touch-panel monitor is used. The display unit 37 displays screens such as a setup screen for the ophthalmic device 10, a manipulation screen (User Interface (UI) screen) for the ophthalmic device 10, anterior eye part images of the subject eye E photographed by the stereo camera 34, a result (a fundus image and a tomographic image of the subject eye E) of examining the subject eye E by the examination head 22.

Examples of the manipulation unit 38 include a publicly known manipulation lever, switches and a manipulation screen to be displayed on the display unit 37 (all not illustrated). The manipulation unit 38 is used to input an instruction such as a positional adjustment of the chin rest 14a and the forehead rest 14b, an XYZ movement and a rotation of the examination head 22, selecting the type of examination (from fundus imaging and OCT imaging), switching between automatic alignment and manual alignment, an examination start, or saving an examination result (a fundus image or a tomographic image).

The storage unit 39 is a recording medium (storage medium) which stores a program to be executed by the control device 40, and various publicly known storages are used as the storage unit 39. A fundus image of the subject eye E photographed by the fundus camera unit 24 and a tomographic image of the subject eye E obtained through OCT imaging by the OCT unit 26 are saved in the storage unit 39.

The control device 40 performs overall control on the action of the units of the ophthalmic device 10 and executes the control such as alignment of the examination head 22 with the subject eye E, imaging of the fundus of the subject eye E by the fundus camera unit 24, and OCT imaging of the subject eye E by the OCT unit 26.

The control device 40 includes an arithmetic circuit including various processors and memories. Examples of the various processors include a Central Processing Unit (CPU), a Graphics Processing Unit (GPU), an Application Specific Integrated Circuit (ASIC), programmable logic devices (e.g., a Simple Programmable Logic Devices (SPLD), a Complex Programmable Logic Device (CPLD), and a Field Programmable Gate Arrays (FPGA)). Various functions of the control device 40 may be implemented by one processor or may be implemented by a plurality of processors of the same type or of different types.

The control device 40 functions as a nose photographing controlling unit 41, a nose position detecting unit 42, a tilting angle determining unit 43, an alignment detection unit 44, a drive controlling unit 46, a vision fixation controlling unit 48, a measurement controlling unit 50, and a saving controlling unit 52 by executing a control program stored in the storage unit 39.

The nose photographing controlling unit 41, the nose position detecting unit 42, and the tilting angle determining unit 43 operate before the start of automatic alignment of the examination head 22 to determine a tilting angle θ (see Figure 5) , at which the examination head 22 is brought closer to the subject eye E from an oblique direction when viewed from the one-direction side in the Y direction at the time of the automatic alignment.

Figure 5 is an explanatory diagram for explaining the automatic alignment of the examination head 22. Here, reference character "OD" in Figure 5 denotes a right eye (oculus dexter) and that reference character "OS" denotes a left eye (oculus sinister). As described earlier, in the ophthalmic device 10 (the fundus camera and the OCT device) supporting wide-angle photographing, an operating distance of the examination head 22 is short due to the objective lens 30 of large size. For this reason, as indicated by reference character 5A in Figure 5, when the examination head 22 is moved from a position in front of the subject eye E (the left eye OS here) toward the front side in the Z direction at the time of the automatic alignment of the examination head 22, the examination head 22 may come closer to the nose N of the subject.

For the above-described reason, as indicated by reference character 5B in Figure 5, when viewed from the one-direction side in the Y direction, the ophthalmic device 10 according to the present embodiment brings the examination head 22 closer to the subject eye E from an oblique direction during the automatic alignment of the examination head 22.

Specifically, assume that an axis along the eye direction (an eye direction of the subject eye E observing infinite distance) of the subject eye E parallel to the Z direction is a reference axis VA, that for X direction, the outward direction away from the nose (N), based on the subject eye E (here, the left eye OS), is defined as X1. Furthermore, an axis obtained by tilting the reference axis VA in the outward direction X1 around the subject eye E is designated as an axis TA of tilt. The examination head 22 is displaced to an examination position where examination (fundus imaging and OCT imaging) of the subject eye E is executable (hereinafter simply referred to as the examination position) along the axis TA of tilt when viewed from the one-direction side in the Y direction. The term "displacement" here subsumes movement in the X, Y, and Z directions and rotation of the examination head 22.

The nose photographing controlling unit 41, the nose position detecting unit 42, and the tilting angle determining unit 43 execute the determination of the tilting angle θ of the axis TA of tilt with respect to the reference axis VA when viewed from the one-direction side in the Y direction, i.e., the tilting angle θ of the axis TA of tilt with respect to the reference axis VA in an XZ plane under control thereof. Specifically, the tilting angle determining unit 43 determines the tilting angle θ through photographing control of the nose N using the stereo camera 34 by the nose photographing controlling unit 41 and position detection of the nose N by the nose position detecting unit 42.

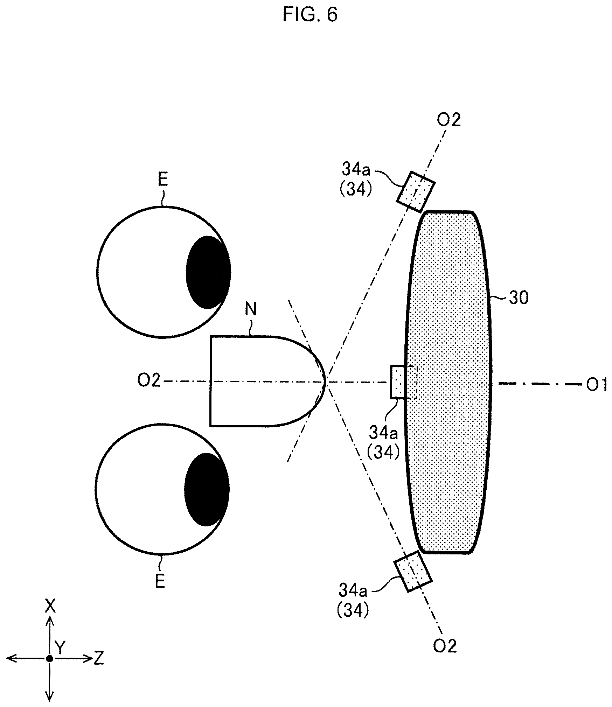

Figure 6 is an explanatory diagram for explaining photographing control of the nose N using the stereo camera 34 by the nose photographing controlling unit 41 and is a diagram of the objective lens 30 and the stereo camera 34 as viewed from an upper side in the Y direction. Here, reference character O2 in Figure 6 denotes a photographing optical axis of each camera 34a.

As illustrated in Figure 6 and Figure 4, the nose photographing controlling unit 41 drives the XZ movement mechanism 16 and the Y movement mechanism 18 to move the examination head 22 to a photographing position where the nose N is photographable by all (three here) cameras 34a before the start of the automatic alignment of the examination head 22, for example, when the examiner manipulates starting examination on the subject E with the manipulation unit 38.

For example, the nose photographing controlling unit 41 estimates three-dimensional position coordinates of the nose N of the subject based on positions in the Y direction of the chin rest 14a and the forehead rest 14b. Alternatively, the nose photographing controlling unit 41 causes all the cameras 34a to execute photographing, and analyzes respective photographed images from the cameras 34a and detects positions of facial parts (e.g., the subject eye E, the nose N, a mouth, and eyebrows) of the subject, thereby estimating the three-dimensional position coordinates of the nose N. The nose photographing controlling unit 41 then detects a simultaneous photographing position which is a photographing position where the nose N is photographable by all the cameras 34a (hereinafter simply referred to as a simultaneous photographing position) based on a result of the estimation of the three-dimensional position coordinates of the nose N and known photographing conditions (a photographic angle of view, and a direction of the photographing optical axis O2) of each camera 34a.

When the nose photographing controlling unit 41 is successful in detecting a simultaneous photographing position, the nose photographing controlling unit 41 positionally adjusts the examination head 22 to the simultaneous photographing position with the XZ movement mechanism 16 and the Y movement mechanism 18. The positional adjustment here includes not moving the examination head 22 when the nose N is photographable by all the cameras 34a at an initial position for the examination head 22 when the ophthalmic device 10 is turned on.

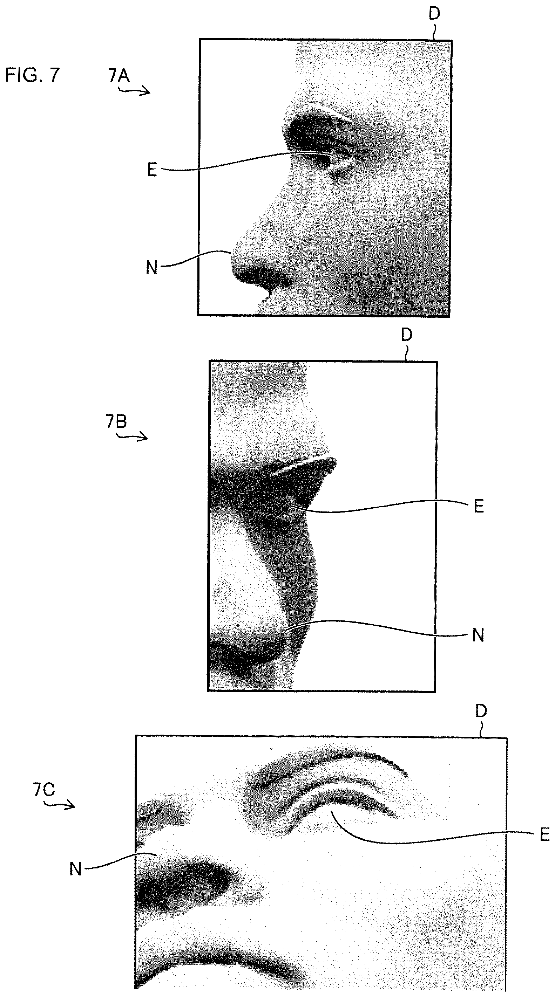

Figure 7 is an explanatory view for explaining photographed nose images D photographed by the three cameras 34a. As illustrated in Figure 7, the nose photographing controlling unit 41 causes all the cameras 34a to simultaneously photograph the nose N after positional adjustment of the examination head 22 and each camera 34a to output the photographed nose image D that is a photographed image of the nose N to the nose position detecting unit 42.

The photographed nose images D indicated by reference characters 7A and 7B in Figure 7 are images photographed by the two cameras 34a provided at the two end portions in the X direction (the two left and right end portions) of the lens-barrel distal end face 28a. The photographed nose image D indicated by reference character 7C in Figure 7 is an image photographed by the camera 34a provided at the lower end portion of the lens-barrel distal end face 28a. Each photographed nose image D is analyzed, and a nose contour is identified from the background (see reference character 7A), a cheek contour of the subject is identified from the background (see reference character 7B), or a nostril is identified (see reference character 7C), thereby allowing identification of an image of a tip of the nose N in the photographed nose image D.

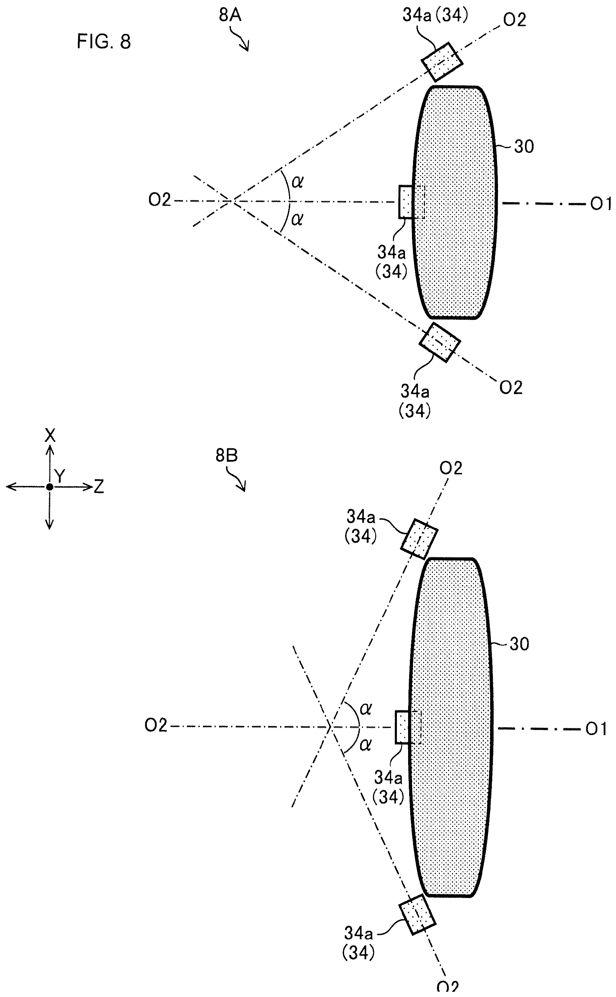

Figure 8 is an explanatory diagram for explaining a case where the nose N is not simultaneously photographable by the three cameras 34a and is a diagram of the objective lens 30 and the stereo camera 34 as viewed from the upper side in the Y direction. The objective lens 30 supporting wide-angle photographing as indicated by reference character 8B in Figure 8 is larger due to optical constraints of the ophthalmic device 10 than the objective lens 30 with a photographic angle of view of about 45° as indicated by reference character 8A in Figure 8. For this reason, when the examination head 22 includes the objective lens 30 supporting wide-angle photographing, an angle α of the photographing optical axis O2 of each camera 34a with respect to the optical axis O1 is large. If photographing of the nose N by all the cameras 34a is impossible unless the examination head 22 is brought closer to the nose N. As a result, the nose photographing controlling unit 41 may be incapable of detecting a simultaneous photographing position or movement of the examination head 22 to a simultaneous photographing position may be impossible.

In such a case, the nose photographing controlling unit 41 executes photographing of the nose N by all the cameras 34a in a non-simultaneous manner in a plurality of batches.

Specifically, the nose photographing controlling unit 41 drives the XZ movement mechanism 16 and the Y movement mechanism 18 to positionally adjust the examination head 22 to a divisional photographing position which is a photographing position where the nose N is photographable by one or more (at least one) cameras 34a (hereinafter referred to as a divisional photographing position) and then execute a divisional photographing process of causing the one or more cameras 34a to photograph the nose N. Simultaneously, the nose photographing controlling unit 41 executes a position information acquiring process of acquiring photographing position information (three-dimensional coordinates) of each of the one or more cameras 34a that have photographed the nose N in the divisional photographing process based on the three-dimensional position coordinates of the examination head 22 at the time of execution of the divisional photographing process and known relative position information of each camera 34a to the examination head 22.

The nose photographing controlling unit 41 repeatedly executes the divisional photographing process and the position information acquiring process until photographing of the nose N by all the cameras 34a is completed. With this process, respective photographed nose images D from the cameras 34a are obtained, and respective pieces of photographing position information of the cameras 34a are obtained, as illustrated in Figure 7.

Referring back to Figure 4, if the nose N is simultaneously photographed by all the cameras 34a, the nose position detecting unit 42 detects relative position information (e.g., three-dimensional coordinates) indicating relative positions in the X, Y, and Z directions of the nose N (e.g., a tip) to the examination head 22 based on respective photographed nose images D photographed by the cameras 34a. Since a method for detecting relative positions of various objects using the stereo camera 34 is a publicly known technique (see, for example, Patent Literature 1), a specific description thereof will be omitted. The nose position detecting unit 42 outputs the relative position information of the nose N to the tilting angle determining unit 43.

On the other hand, if photographing of the nose N by all the cameras 34a is executed in a plurality of batches, the nose position detecting unit 42 detects the relative position information of the nose N to the examination head 22 based on respective photographed nose images D from and respective pieces of photographing position information of the cameras 34a and outputs the relative position information to the tilting angle determining unit 43. Here, the nose position detecting unit 42, for example, converts the respective photographed nose images D from the cameras 34a into images photographed at an identical photographing position based on the respective pieces of photographing position information of the cameras 34a and detects the relative position information of the nose N based on the photographed nose images D after the conversion.

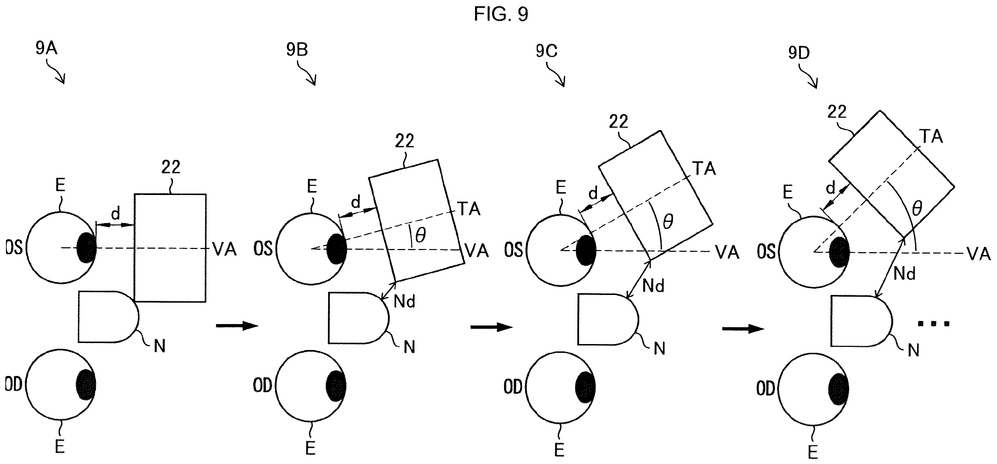

Figure 9 is an explanatory diagram for explaining an example of a method for determining the tilting angle θ by the tilting angle determining unit 43. As illustrated in Figure 9 and Figure 4, the tilting angle determining unit 43 determines the tilting angle θ based on the relative position information of the nose N input from the nose position detecting unit 42.

For example, the tilting angle determining unit 43 computes, for each of a plurality of tilting angles θ different from each other, a nose distance Nd which is a distance (shortest distance) between the examination head 22 and the nose N when the examination head 22 is brought closer to an operating distance d determined in advance from the subject eye E based on the relative position information of the nose N, as indicated by reference characters 9A to 9D in Figure 9. The tilting angle determining unit 43, for example, determines, as the tilting angle θ at the time of the automatic alignment, a smallest tilting angle θ among tilting angles θ, for which nose distances Nd are equal to or more than a predetermined threshold, based on the respective nose distances Nd for the plurality of tilting angles θ.

Referring back to Figure 4, the alignment detection unit 44 executes identification of a pupil center position of the subject eye E and computation of three-dimensional coordinates of the pupil center position based on anterior eye part images of the subject eye E stereoscopically photographed by the cameras 34a of the stereo camera 34 during the automatic alignment of the examination head 22, thereby detecting a relative position of the subject eye E to the examination head 22. Since a method for alignment detection using the stereo camera 34 is a publicly known technique (see Patent Literature 1 described above), a specific description thereof will be omitted.

The drive controlling unit 46 drives the XZ movement mechanism 16, the Y movement mechanism 18, and the swing rotation mechanism 20 to execute alignment of the examination head 22 with the subject eye E and switching of the subject eye E as an examination object (left-right eye switching). The alignment of the examination head 22 includes automatic alignment that is performed by automatically driving the XZ movement mechanism 16, the Y movement mechanism 18, and the swing rotation mechanism 20 and manual alignment that drives the XZ movement mechanism 16, the Y movement mechanism 18, and the swing rotation mechanism 20 in accordance with a manipulation input to the manipulation unit 38. The examiner instructs switching between the automatic alignment and the manual alignment is executed with the manipulation unit 38.

The drive controlling unit 46 determines the axis TA of tilt corresponding to the tilting angle θ based on the tilting angle θ initially determined by the tilting angle determining unit 43 at the time of the automatic alignment.

For example, the drive controlling unit 46 identifies the reference axis VA based on photographed images obtained through initial stereoscopic photographing of the face (e.g., the subject eye E or the nose N) of the subject by the stereo camera 34. Alternatively, the drive controlling unit 46 estimates the reference axis VA based on the positions in the Y direction of the chin rest 14a and the forehead rest 14b and identification information on the left eye OS and the right eye OD that are already known. The drive controlling unit 46 determines, as the axis TA of tilt, an axis obtained by tilting the reference axis VA in the outward direction X1 around the subject eye E by the tilting angle θ.

The drive controlling unit 46 then drives the XZ movement mechanism 16, the Y movement mechanism 18, and the swing rotation mechanism 20 based on the axis TA of tilt to start automatic alignment that automatically displaces the examination head 22 from the initial position before the start of the automatic alignment of the ophthalmic device 10 to the examination position.

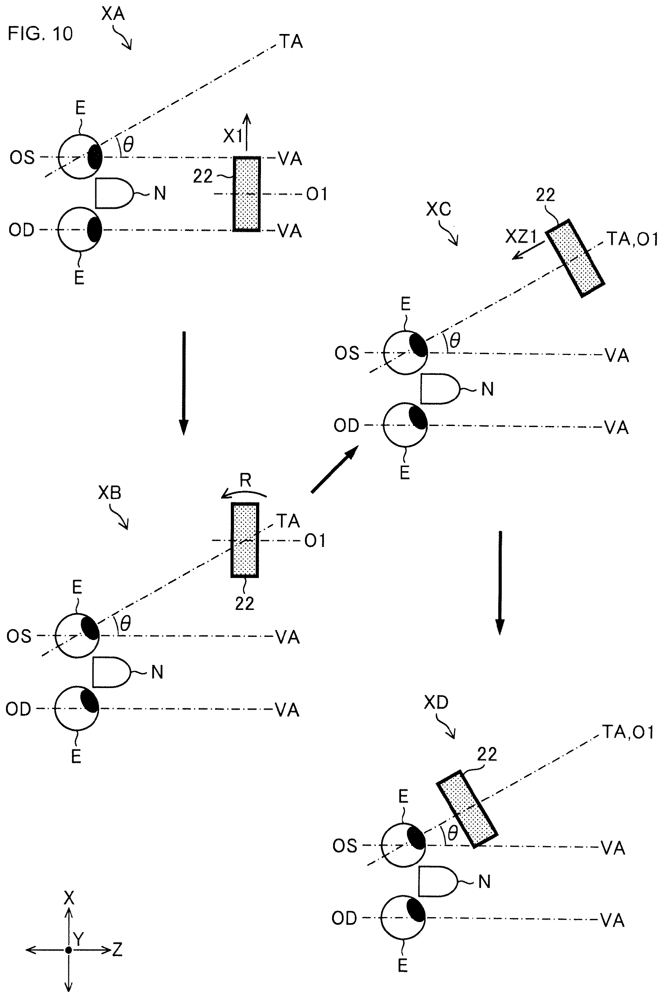

Figure 10 is an explanatory diagram for explaining an example 1-1 of the automatic alignment of the examination head 22 according to the first embodiment. As indicated by reference character XA in Figure 10, the examination head 22 is initially arranged, for example, at a position where the optical axis O1 of the objective lens 30 is between the reference axes VA of the left and right subject eyes E (the left eye OS and the right eye OD) when viewed from the one-direction side in the Y direction, i.e., a position facing (the term "face" as used herein is intended to include the meaning of "substantially face"; the same applies hereinafter) the nose N.

First, the drive controlling unit 46 drives the XZ movement mechanism 16 to execute a first driving process of moving the examination head 22 from the initial position to the axis TA of tilt in the outward direction X1 when the examination head 22 is viewed from the one-direction side in the Y direction.

As indicated by reference character XB in Figure 10, the drive controlling unit 46 drives the swing rotation mechanism 20 after completion of the first driving process to execute a second driving process of rotating the examination head 22 around the rotating shaft 20a by the tilting angle θ (see an arrow R). With this process, as indicated by reference character XC in Figure 10, the optical axis O1 of the examination head 22 (the objective lens 30) becomes parallel to the axis TA of tilt. The second driving process may be executed before the first driving process.

The drive controlling unit 46 then drives the XZ movement mechanism 16 after completion of the second driving process to start a third driving process of moving the examination head 22 to the examination position along the axis TA of tilt when the examination head 22 is viewed from the one-direction side in the Y direction (see an arrow XZ1). With this process, the examination head 22 is moved toward the subject eye E while keeping the tilting angle θ constant (the term "constant" as used herein is intended to include the meaning of "substantially constant"; the same applies hereinafter).

Halfway through the automatic alignment, the examination head 22 is displaced to a position where the alignment detection unit 44 can identify the pupil center position of the subject eye E, i.e., a position where the alignment detection is possible. For this reason, an alignment detection result is input from the alignment detection unit 44 to the drive controlling unit 46. Y-direction positional adjustment of the examination head 22 by the Y movement mechanism 18 may be executed before the alignment detection (at any stage from the first driving process to the third driving process) such that the alignment detection by the alignment detection unit 44 is possible.

As indicated by reference character XD in Figure 10, the drive controlling unit 46 drives the XZ movement mechanism 16 and the Y movement mechanism 18 based on an alignment detection result input from the alignment detection unit 44 to continue the third driving process until the examination head 22 reaches the examination position. In the third driving process that is executed based on the alignment detection result, the Y-direction positional adjustment of the examination head 22 is also executed.

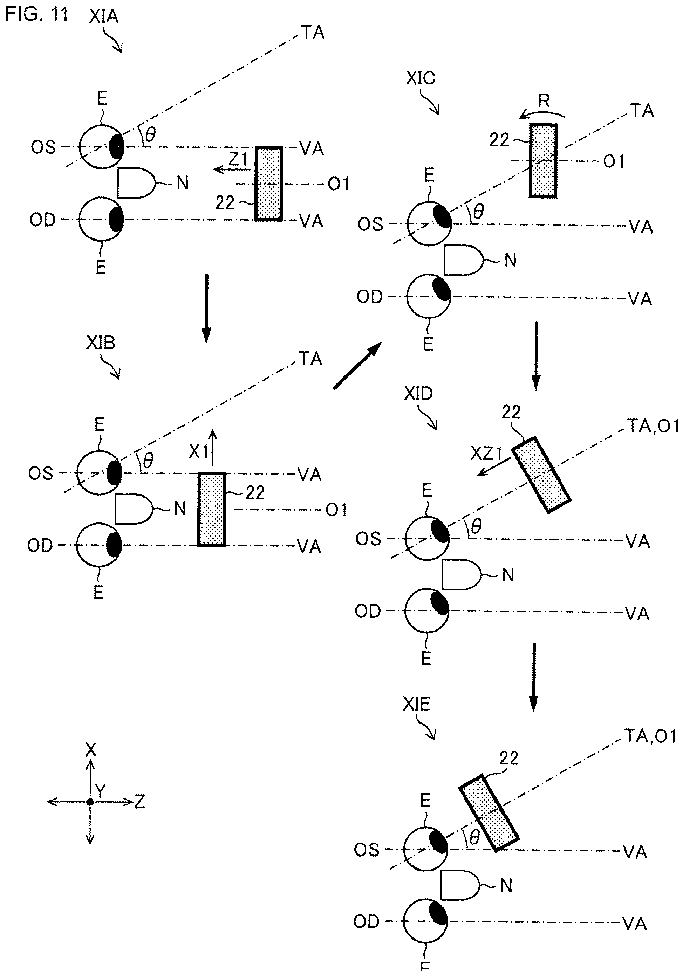

Figure 11 is an explanatory diagram for explaining an example 1-2 of the automatic alignment of the examination head 22 according to the first embodiment. The drive controlling unit 46 drives the XZ movement mechanism 16 to execute a first driving process of first moving the examination head 22 toward the front side (the subject eye E side) in the Z direction by a predetermined distance (see an arrow Z1), as indicated by reference character XIA in Figure 11, and then moving the examination head 22 to the axis TA of tilt in the outward direction X1, as indicated by reference character XIB in Figure 11. Here, a distance of movement of the examination head 22 toward the front side in the Z direction is not particularly limited as long as a safe distance can be secured between the examination head 22 and the nose N. For example, the distance of movement may be determined based on a result of computing a Z-direction distance from the examination head 22 to the nose N based on photographed images obtained through stereoscopic photographing of the nose N by the stereo camera 34.

By first moving the examination head 22 toward the front side in the Z direction and then moving the examination head 22 to the axis TA of tilt in the outward direction X1, a distance of movement, by which the examination head 22 is moved in the outward direction X1, can be made smaller than in the example 1-1 illustrated in Figure 10 described earlier.

As indicated by reference character XIC in Figure 11, the drive controlling unit 46 drives the swing rotation mechanism 20 after completion of the first driving process to execute the same second driving process as the example 1-1 (see reference character XB in Figure 10) and make the optical axis O1 parallel to the axis TA of tilt. The second driving process may be executed before the first driving process in the example 1-2 as well.

As indicated by reference character XID in Figure 11, the drive controlling unit 46 drives the XZ movement mechanism 16 after completion of the second driving process to execute a third driving process in the same manner as the example 1-1 (see reference characters XC and XD in Figure 10), thereby moving the examination head 22 to the examination position along the axis TA of tilt when the examination head 22 is viewed from the one-direction side in the Y direction. As indicated by reference character XIE in Figure 11, the drive controlling unit 46 drives the XZ movement mechanism 16 and the Y movement mechanism 18 based on alignment detection performed by the alignment detection unit 44 halfway through the automatic alignment to continue the third driving process until the examination head 22 reaches the examination position.

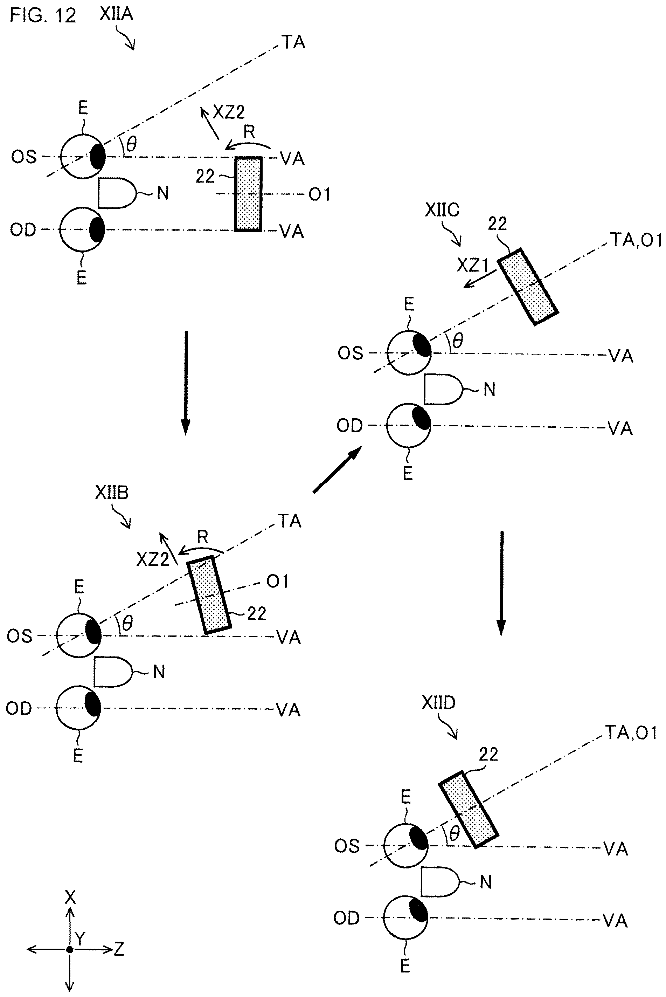

Figure 12 is an explanatory diagram for explaining an example 1-3 of the automatic alignment of the examination head 22 according to the first embodiment. As indicated by reference characters XIIA and XIIB in Figure 12, the drive controlling unit 46 drives the XZ movement mechanism 16, the Y movement mechanism 18, and the swing rotation mechanism 20 to execute a first driving process of simultaneously executing movement of the examination head 22 toward the front side in the Z direction and in the outward direction X1 and rotation of the examination head 22 by the tilting angle θ (see an arrow XZ2 and the arrow R). With this process, it is possible to move the examination head 22 to the axis TA of tilt in an oblique direction when the examination head 22 is viewed from the one-direction side in the Y direction and make the optical axis O1 parallel to the axis TA of tilt.

In the first driving process in the example 1-3, the examination head 22 may be displaced to a position on the axis TA of tilt where the stereo camera 34 can photograph the anterior eye part of the subject eye E or to a position where an observation optical system (not illustrated) inside the examination head 22 can photograph the anterior eye part of the subject eye E via a shortest route.

As indicated by reference character XIIC in Figure 12, the drive controlling unit 46 drives the XZ movement mechanism 16 and the Y movement mechanism 18 after completion of the first driving process to execute a second driving process. The second driving process in the example 1-3 is the same process as the third driving processes in the example 1-1 and the example 1-2, and moves the examination head 22 to the examination position along the axis TA of tilt when the examination head 22 is viewed from the one-direction side in the Y direction. As indicated by reference character XIID in Figure 12, the drive controlling unit 46 drives the XZ movement mechanism 16 and the Y movement mechanism 18 based on alignment detection performed by the alignment detection unit 44 halfway through the automatic alignment to continue the second driving process until the examination head 22 reaches the examination position.

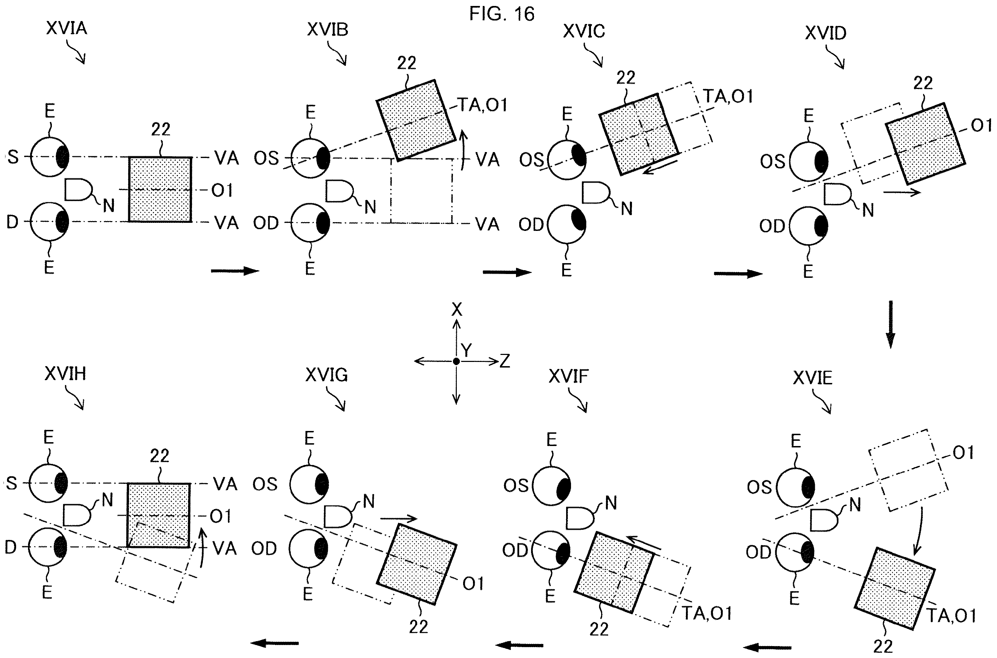

Referring back to Figure 4, when examination on the subject eye E by the examination head 22 (fundus imaging of the subject eye E by the fundus camera unit 24 or OCT imaging of the subject eye E by the OCT unit 26) is completed, the drive controlling unit 46 drives the XZ movement mechanism 16 to retract the examination head 22 toward a rear side in the Z direction (an examiner side) by a predetermined distance (see reference characters XVID and XVIG in Figure 16 (to be described later)).

The vision fixation controlling unit 48 causes the vision fixation light emitting unit 36 to emit vision fixation light at least during a period from before the start of the automatic alignment of the examination head 22 to completion of examination on the subject eye E by the examination head 22. This makes it possible to guide and fix an eye direction of the subject to a direction of the vision fixation light during movement of the examination head 22 from the initial position to the examination position through the axis TA of tilt at the time of the automatic alignment. For this reason, for example, when the ophthalmic device 10 moves the examination head 22 from the initial position to the axis TA of tilt, the subject eye E can be made to circumnutate so as to follow the movement (see Figures 10 to 12). As a result, the eye direction of the subject eye E can be kept fixed to the examination head 22.

The measurement controlling unit 50 controls fundus imaging of the subject eye E by the fundus camera unit 24 and photographing of a tomographic image of the subject eye E by the OCT unit 26. For example, the measurement controlling unit 50 drives a focus optical system (not illustrated) (see Patent Literature 1) housed in the examination head 22 after completion of the automatic alignment of the examination head 22 to execute an autofocusing process of focusing the examination head 22 on a part to be observed (e.g., the fundus) of the subject eye E. The measurement controlling unit 50 then executes fundus imaging of the subject eye E by the fundus camera unit 24 or OCT imaging of the subject eye E by the OCT unit 26.

The measurement controlling unit 50 acquires a fundus image of the subject eye E from the fundus camera unit 24 and outputs the fundus image to the saving controlling unit 52 when fundus imaging of the subject eye E by the fundus camera unit 24 is executed. The measurement controlling unit 50 generates a tomographic image of the subject eye E based on a signal such as a detection signal output from the OCT unit 26 by a publicly known method and outputs the tomographic image to the saving controlling unit 52 when OCT imaging of the subject eye E by the OCT unit 26 is executed.

The saving controlling unit 52 causes the display unit 37 to display a fundus image or a tomographic image of the subject eye E input from the measurement controlling unit 50. The saving controlling unit 52 saves the fundus image or the tomographic image of the subject eye E in the storage unit 39 when an examiner inputs an image saving to the manipulation unit 38.

### [Function of Ophthalmic Device According to First Embodiment]

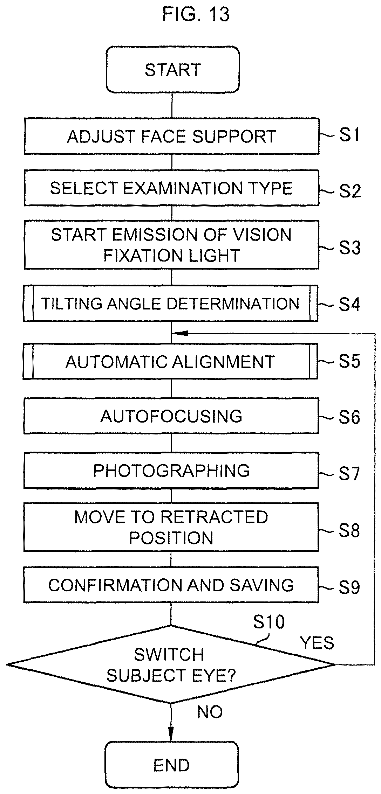

Figure 13 is a flowchart illustrating a flow of a process of examining the subject eye E by the ophthalmic device 10 according to the first embodiment with the above-described configuration. As illustrated in Figure 13, the examiner manipulates the manipulation unit 38 with the subject placing his/her chin against the chin rest 14a and his/her forehead against the forehead rest 14b to adjust height positions (the positions in the Y direction) of the chin rest 14a and the forehead rest 14b to fit the subject (step S1).

The examiner then manipulates the manipulation unit 38 to select the type of examination on the subject eye E (fundus imaging by the fundus camera unit 24 or OCT imaging by the OCT unit 26) (step S2). The examiner also manipulates the manipulation unit 38 to select an automatic alignment mode as an alignment mode of the examination head 22. When the examination type of the subject eye E and the alignment mode are selected, the vision fixation controlling unit 48 causes the vision fixation light emitting unit 36 to emit vision fixation light (step S3). This allows guiding and fixation of the eye direction of the subject eye E.

When the examiner inputs an examination start to the manipulation unit 38, the nose photographing controlling unit 41, the nose position detecting unit 42, and the tilting angle determining unit 43 first operate to start a process of determining the tilting angle θ (step S4).

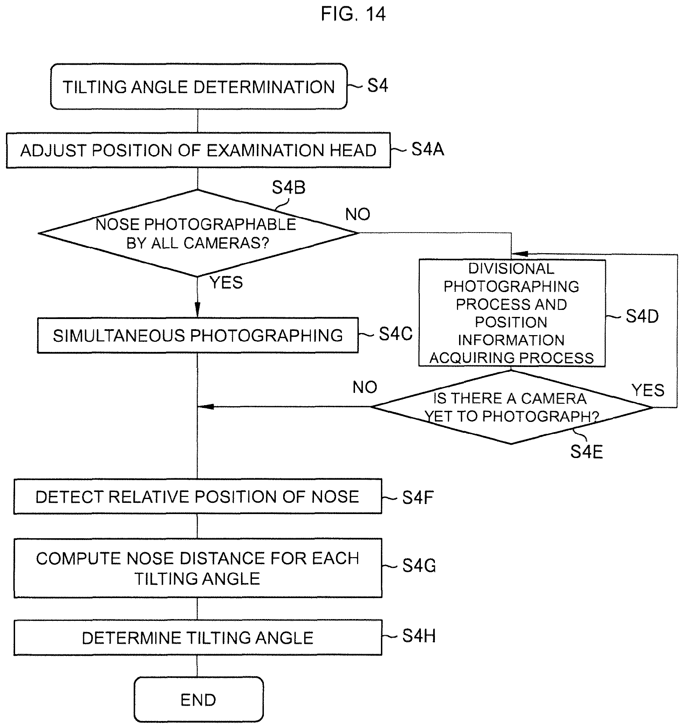

Figure 14 is a flowchart illustrating a flow of the process of determining the tilting angle θ which pertains to a method for operating an ophthalmic device according to the presently disclosed subject matter. As illustrated in Figure 14, the nose photographing controlling unit 41 drives the XZ movement mechanism 16 and the Y movement mechanism 18 to execute positional adjustment of the examination head 22 (step S4A).

At this time, the nose photographing controlling unit 41 estimates the three-dimensional position coordinates of the nose N of the subject based on the positions in the Y direction of the chin rest 14a and the forehead rest 14b or estimates the three-dimensional position coordinates of the nose N of the subject by analyzing photographed images obtained through photographing of the face of the subject by the cameras 34a. The nose photographing controlling unit 41 then executes detection of a simultaneous photographing position based on the result of estimating the three-dimensional position coordinates of the nose N and the known photographing conditions for the cameras 34a.

When the nose photographing controlling unit 41 is successful in detecting a simultaneous photographing position to which the examination head 22 can be moved, the nose photographing controlling unit 41 positionally adjusts the examination head 22 to the simultaneous photographing position with the XZ movement mechanism 16 and the Y movement mechanism 18 (YES in step S4B). The nose photographing controlling unit 41 causes all the cameras 34a to simultaneously photograph the nose N and each camera 34a to output a photographed nose image D to the nose position detecting unit 42 (step S4C corresponding to a nose photographing controlling step according to the presently disclosed subject matter).

On the other hand, when the nose photographing controlling unit 41 is unsuccessful in detecting a simultaneous photographing position to which the examination head 22 can be moved (NO in step S4B), the nose photographing controlling unit 41 drives the XZ movement mechanism 16 and the Y movement mechanism 18 to positionally adjust the examination head 22 to a divisional photographing position and then executes the divisional photographing process of causing one or more of the cameras 34a to photograph the nose N (step S4D). Simultaneously, the nose photographing controlling unit 41 executes the position information acquiring process (step S4D) of acquiring photographing position information of each of the one or more cameras 34a that have photographed the nose N in the divisional photographing process.

After that, the nose photographing controlling unit 41 executes a repetition process of repeating the divisional photographing process and the position information acquiring process described above until photographing of the nose N by all the cameras 34a is completed (YES in step S4E and step S4D, corresponding to the nose photographing controlling step according to the presently disclosed subject matter). With this process, the photographing of the nose N by all the cameras 34a is executed in a plurality of batches, and respective photographed nose images D from and respective pieces of photographing position information of the cameras 34a are output to the nose position detecting unit 42 (NO in step S4E).

When the nose N is simultaneously photographed by all the cameras 34a, the nose position detecting unit 42 detects the relative position information of the nose N to the examination head 22 based on the respective photographed nose images D photographed by the cameras 34a and outputs the relative position information to the tilting angle determining unit 43 (step S4F). When photographing of the nose N by all the cameras 34a is executed in a plurality of batches, the nose position detecting unit 42 detects the relative position information of the nose N based on the respective photographed nose images D from and the respective pieces of photographing position information of the cameras 34a and outputs the relative position information to the tilting angle determining unit 43 (step S4F). Here, step S4F corresponds to a nose position detecting step according to the presently disclosed subject matter.

The tilting angle determining unit 43 then computes, for each of a plurality of tilting angles θ different from each other, a nose distance Nd based on the relative position information of the nose N (step S4G), as illustrated in Figure 9. The tilting angle determining unit 43, for example, determines, as the tilting angle θ at the time of the automatic alignment, a smallest tilting angle θ among tilting angles θ, for which nose distances Nd are equal to or more than the predetermined threshold, based on a result of computing the nose distances Nd for the plurality of tilting angles θ (step S4H, corresponding to a tilting angle determining step according to the presently disclosed subject matter). This makes it possible to minimize the amount of movement of the examination head 22 while preventing the examination head 22 at the time of the automatic alignment from coming closer to the nose N.

Referring back to Figure 13, when the determination of the tilting angle θ by the tilting angle determining unit 43 is completed, the automatic alignment of the examination head 22 with the subject eye E is executed (step S5).

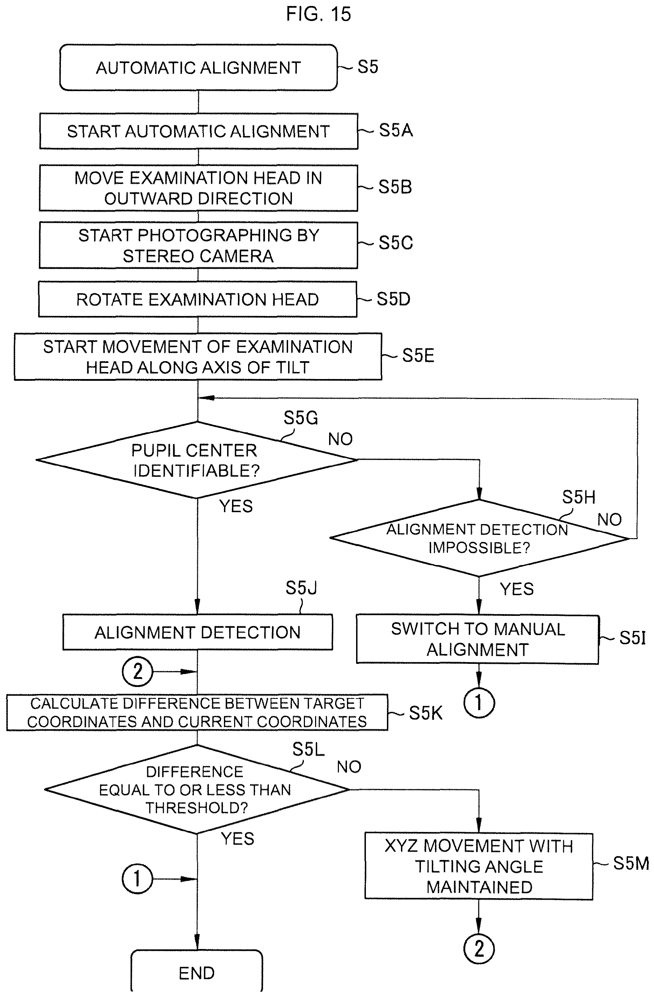

Figure 15 is a flowchart illustrating a flow of an automatic alignment process for the examination head 22 which pertains to the operation method for an ophthalmic device according to the presently disclosed subject matter. Figure 16 is an explanatory diagram for explaining displacement of the examination head 22 after the start of the automatic alignment. The subject eye E is the left eye OS here. And, an example of the automatic alignment in the example 1-1 illustrated in Figure 10 will be described below.

As illustrated in Figures 15 and 16, after determination of the axis TA of tilt based on the tilting angle θ determined by the tilting angle determining unit 43, the drive controlling unit 46 starts the automatic alignment of the examination head 22 (step S5A).

The drive controlling unit 46 drives the XZ movement mechanism 16 to first execute a first driving process of moving the examination head 22 from the initial position to the axis TA of tilt in the outward direction X1 when the examination head 22 is viewed from the one-direction side in the Y direction (step S5B). The alignment detection unit 44 causes the cameras 34a of the stereo camera 34 to start photographing and continuously executes acquisition of photographed images from the cameras 34a and analysis of the photographed images (step S5C).

The drive controlling unit 46 then drives the swing rotation mechanism 20 to execute a second driving process of rotating the examination head 22 around the rotating shaft 20a by the tilting angle θ and making the optical axis O1 of the examination head 22 parallel to the axis TA of tilt (step S5D). With this process, the examination head 22 is displaced from the initial position indicated by reference character XVIA in Figure 16 to the axis TA of tilt, as indicated by reference character XVIB, and the optical axis O1 becomes parallel to the axis TA of tilt. Emission of vision fixation light from the vision fixation light emitting unit 36 allows the eye direction of the subject eye E to follow displacement of the examination head 22.

The drive controlling unit 46 drives the XZ movement mechanism 16 to start a third driving process of moving the examination head 22 to the examination position along the axis TA of tilt when the examination head 22 is viewed from the one-direction side in the Y direction (step S5E), as indicated by reference character XVIC in Figure 16.

While the first driving process to the third driving process are executed, the alignment detection unit 44 waits for a chance for alignment detection (NO in step S5G and NO in step S5H) until the pupil center position of the subject eye E can be identified from photographed images acquired from the cameras 34a.

The drive controlling unit 46 determines that alignment detection is impossible (NO in step S5G and YES in step S5H) when the alignment detection unit 44 is incapable of identifying the pupil center position of the subject eye E during a period from the start of the automatic alignment or the start of the third driving process to movement of the examination head 22 for a fixed time determined in advance or over a fixed distance. In this case, the drive controlling unit 46 switches the alignment mode of the examination head 22 from the automatic alignment mode to a manual alignment mode and displays a message to that effect on the display unit 37 (step S5I). Based on the message, the examiner manipulates the manipulation unit 38 to execute manual alignment of the examination head 22. Since a switch to the manual alignment can be made halfway through the automatic alignment, the examination head 22 is prevented from coming closer to the subject eye E in a state where alignment detection is impossible.

When the cameras 34a photograph the anterior eye part of the subject eye E halfway through the automatic alignment, the alignment detection unit 44 is capable of identifying the pupil center position of the subject eye E based on photographed images input from the cameras 34a to the alignment detection unit 44, i.e., anterior eye part images of the subject eye E (YES in step S5G).

The alignment detection unit 44 then executes alignment detection that detects the relative position of the subject eye E to the examination head 22 by converting the pupil center position of the subject eye E into three-dimensional coordinates (step S5J). The alignment detection unit 44 outputs a detection result of the alignment detection to the drive controlling unit 46.

The drive controlling unit 46 drives the XZ movement mechanism 16 and the Y movement mechanism 18 based on the result of the alignment detection input from the alignment detection unit 44 to continue the third driving process until the examination head 22 reaches the examination position. Specifically, the drive controlling unit 46 calculates a difference between three-dimensional coordinates (target coordinates) of the examination position determined based on the alignment detection result and three-dimensional coordinates of the current examination head 22 (current coordinates) and continues the third driving process until the difference is equal to or less than a threshold (step S5K, NO in step S5L, and step S5M). In this manner, the examination head 22 is moved to the examination position while maintaining the tilting angle θ (corresponding to a drive controlling step according to the presently disclosed subject matter).

When the difference between the target coordinates and the current coordinates is equal to or less than the threshold, the drive controlling unit 46 stops driving the XZ movement mechanism 16 and the Y movement mechanism 18 to end the automatic alignment (YES in step S5L). The above-described displacement of the examination head 22 to the examination position along the axis TA of tilt at the time of the automatic alignment prevents the examination head 22 from coming closer to the nose N.

Referring back to Figures 13 and 16, when the automatic alignment is completed, the measurement controlling unit 50 drives the focus optical system (not illustrated) to execute autofocusing (step S6). After that, the measurement controlling unit 50 executes fundus imaging of the subject eye E by the fundus camera unit 24 or OCT imaging of the subject eye E by the OCT unit 26 (step S7). The measurement controlling unit 50 outputs a fundus image of the subject eye E acquired from the fundus camera unit 24 to the saving controlling unit 52 when fundus imaging is executed. The measurement controlling unit 50 generates a tomographic image of the subject eye E based on a signal such as a detection signal output from the OCT unit 26 and outputs the tomographic image to the saving controlling unit 52 when OCT imaging is executed.

When fundus imaging or OCT imaging of the subject eye E is completed, the drive controlling unit 46 drives the XZ movement mechanism 16 to retract the examination head 22 toward the rear side in the Z direction (step S8), as indicated by reference character XV1D in Figure 16.

The saving controlling unit 52 causes the display unit 37 to display the fundus image or the tomographic image of the subject eye E input from the measurement controlling unit 50. This allows the examiner to confirm whether a desired fundus image or tomographic image is obtained. When a desired fundus image or tomographic image is obtained, the examiner inputs an image saving to the manipulation unit 38. With this manipulation, the saving controlling unit 52 saves the fundus image or tomographic image of the subject eye E in the storage unit 39 (step S9).

When the examiner proceeds to examine the right eye OD, the processes in step S5 to step S9 are repeatedly executed (YES in step S10). In this case, the examination head 22 is displaced to the axis TA of tilt corresponding to the right eye OD (see reference character XV1E in Figure 16) and is further moved to an examination position for the right eye OD along the axis TA of tilt (see reference character XV1F in Figure 16), under control by the drive controlling unit 46. When the examination on the right eye OD is completed, the examination head 22 is retracted toward the rear side in the Z direction (see reference character XV1G in Figure 16) and is then displaced to the initial position (see reference character XV1H in Figure 16), under the control by the drive controlling unit 46.

As described above, in the ophthalmic device 10 according to the first embodiment, the tilting angle θ can be determined based on photographed nose images D obtained through photographing of the nose N by the stereo camera 34, and the examination head 22 can be moved to the examination position for the subject eye E along the axis TA of tilt tilted at the tilting angle θ at the time of the automatic alignment. For this reason, a sufficient distance can always be secured between the examination head 22 and the nose N as compared to a case where the examination head 22 is moved from a position in front of the subject eye E to an examination position on the front side in the Z direction (see reference character 5A in Figure 5). As a result, it is possible to examine the subject eye E by the examination head 22 without bringing the examination head 22 closer to the nose N.

### [Second Embodiment]

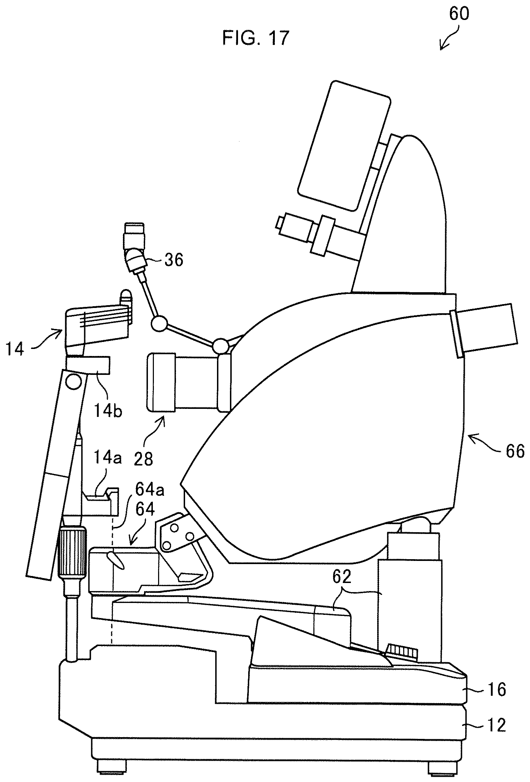

Figure 17 is a side view of an ophthalmic device 60 according to a second embodiment. While the examination head 22 is rotated (swung) around the objective lens 30 by the swing rotation mechanism 20 (the rotating shaft 20a) in the ophthalmic device 10 according to the above-described first embodiment, the ophthalmic device 60 according to the second embodiment includes an examination head 66 different in rotation center position from that in the first embodiment.

Components functionally or structurally identical to those in the ophthalmic device 10 according to the first embodiment are denoted by identical reference numerals, and a description thereof will be omitted.

As illustrated in Figure 17, the ophthalmic device 60 is a fundus camera and includes a base 12, a face support 14, an XZ movement mechanism 16, a vision fixation light emitting unit 36 (only an external fixation lamp of which is illustrated), a Y movement mechanism 62, a swing rotation mechanism 64, and the examination head 66. The ophthalmic device 60 also includes a stereo camera 34, a display unit 37, a manipulation unit 38, a storage unit 39, and a control device 40 (all not illustrated) described in the first embodiment.

The Y movement mechanism 62, together with the XZ movement mechanism 16, constitutes a movement mechanism according to the presently disclosed subject matter. The Y movement mechanism 62 has a shape extending toward a front side in a Z direction. The swing rotation mechanism 64 is provided at a distal end portion on the front side in the Z direction of the Y movement mechanism 62. The Y movement mechanism 62 integrally moves the swing rotation mechanism 64 and the examination head 66 in a Y direction. The XZ movement mechanism 16 and the Y movement mechanism 62 are capable of integrally moving the swing rotation mechanism 64 and the examination head 66 in an X direction and the Y and Z directions.

The swing rotation mechanism 64 corresponds to a rotation mechanism according to the presently disclosed subject matter and, together with the XZ movement mechanism 16 and the Y movement mechanism 62, constitutes a displacement mechanism according to the presently disclosed subject matter. The swing rotation mechanism 64 has a rotation axis 64a parallel to the Y direction and rotates the examination head 66 around the rotation axis 64a. The rotation axis 64a is provided in front of a lens-barrel 28 (an objective lens 30) of the examination head 66 in the Z direction. With this configuration, the rotation axis 64a and a subject eye E (a circumnutation center) can be made to coincide when viewed from a one-direction side in the Y direction by adjusting X and Z positions of the swing rotation mechanism 64 by the XZ movement mechanism 16. In this case, the swing rotation mechanism 64 rotates (swings) the examination head 66 around the circumnutation center of the subject eye E.

Although not illustrated, the swing rotation mechanism 64 can also rotate (tilt) the examination head 66 around a rotation axis perpendicular to the Y direction.

The examination head 66 is attached to the swing rotation mechanism 64. With this configuration, the examination head 66 is movable in the X, Y, and Z directions by the XZ movement mechanism 16 and the Y movement mechanism 62 and is rotatable in a direction around the rotation axis 64a by the swing rotation mechanism 64. The examination head 66 includes a fundus camera unit 24 and the lens-barrel 28 (including the stereo camera 34) described in the first embodiment.

The control device 40 according to the second embodiment is basically the same as the control device 40 according to the first embodiment except that a method for automatic alignment of the examination head 66 by a drive controlling unit 46 is different.

The drive controlling unit 46 according to the second embodiment determines an axis TA of tilt based on a tilting angle θ determined by a tilting angle determining unit 43 and then drives the XZ movement mechanism 16, the Y movement mechanism 62, and the swing rotation mechanism 64 to execute the automatic alignment of the examination head 66, like the first embodiment.

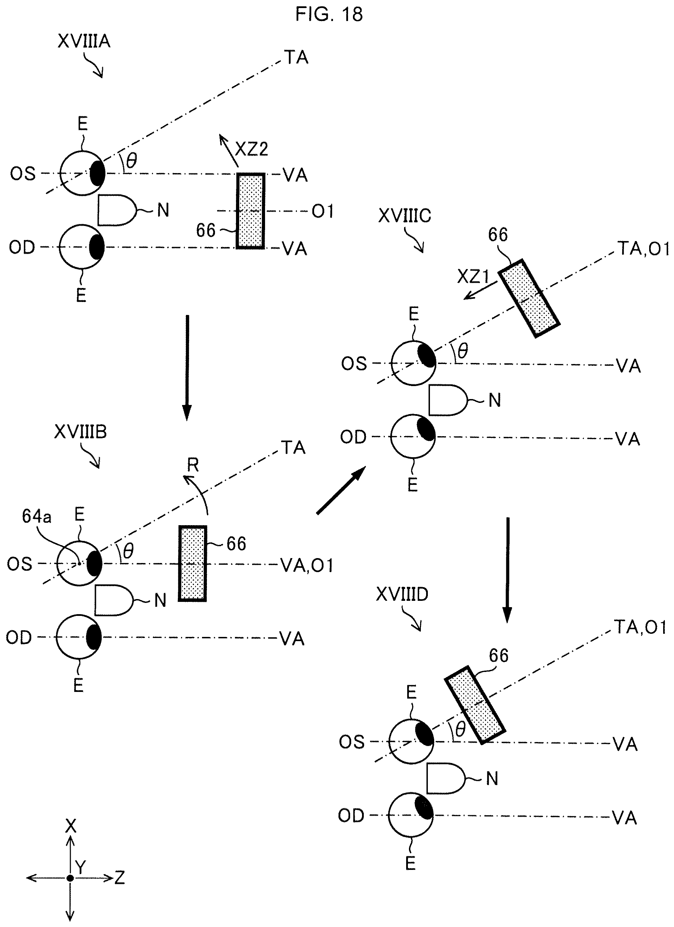

Figure 18 is an explanatory diagram for explaining an example 2-1 of the automatic alignment of the examination head 66 according to the second embodiment. As indicated by reference character XVIIIA in Figure 18, the examination head 66 is arranged at the same initial position as the first embodiment at first.

As indicated by reference characters XVIIIA and XVIIIB in Figure 18, the drive controlling unit 46 drives the XZ movement mechanism 16 to execute a first driving process of moving the examination head 66 (the swing rotation mechanism 64) from the initial position in the X and Z directions (see an arrow XZ2). Specifically, the examination head 66 is moved in the X and Z directions to a position where the rotation axis 64a coincides with the circumnutation center of the subject eye E when viewed from the one-direction side in the Y direction.

The drive controlling unit 46 drives the swing rotation mechanism 20 after completion of the first driving process to execute a second driving process of rotating the examination head 66 around the rotating shaft 20a (the circumnutation center of the subject eye E) by the tilting angle θ (see an arrow R). With this process, as indicated by reference character XVIIIC in Figure 18, the examination head 66 is moved to the axis TA of tilt, and an optical axis O1 becomes parallel to the axis TA of tilt.

The drive controlling unit 46 then drives the XZ movement mechanism 16 after completion of the second driving process to start a third driving process of moving the examination head 66 to an examination position along the axis TA of tilt when the examination head 66 is viewed from the one-direction side in the Y direction (see an arrow XZ1), like the first embodiment. In the third driving process according to the second embodiment, Z-axis movement of the examination head 66 by the XZ movement mechanism 16 is mainly executed, unlike the first embodiment. With this movement, the examination head 66 is moved toward the subject eye E while keeping the tilting angle θ constant.

As indicated by reference character XVIIID in Figure 18, the drive controlling unit 46 drives the XZ movement mechanism 16 and the Y movement mechanism 62 based on alignment detection performed by an alignment detection unit 44 halfway through the automatic alignment to continue the third driving process until the examination head 66 reaches the examination position.

The drive controlling unit 46 switches an alignment mode of the examination head 66 to a manual alignment mode when the alignment detection unit 44 is incapable of alignment detection during a period from the start of the automatic alignment or the start of the third driving process to movement of the examination head 66 for a fixed time determined in advance or over a fixed distance, like the first embodiment.

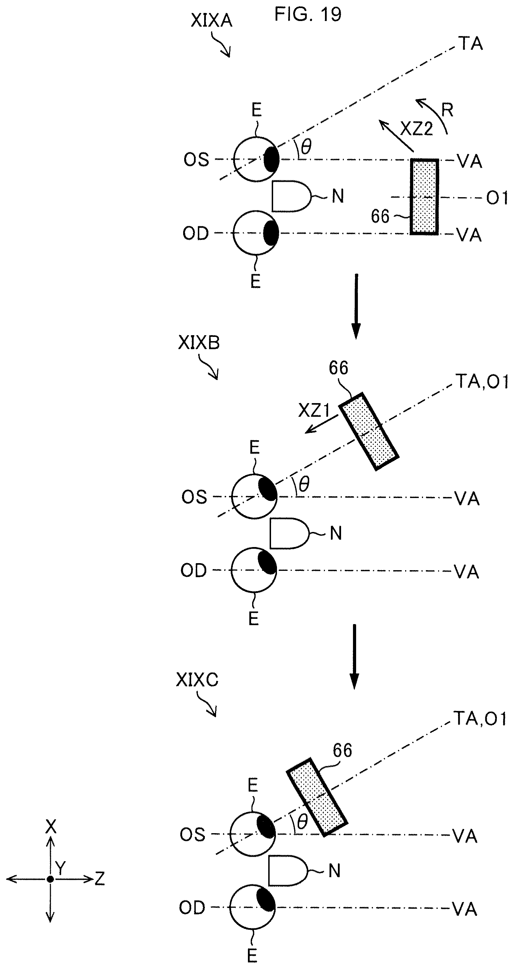

Figure 19 is an explanatory diagram for explaining an example 2-2 of the automatic alignment of the examination head 66 according to the second embodiment. As indicated by reference characters XIXA and XIXB in Figure 19, the drive controlling unit 46 simultaneously drives the XZ movement mechanism 16, the Y movement mechanism 62, and the swing rotation mechanism 64 to execute a first driving process of simultaneously executing movement of the examination head 66 in the X and Z directions and rotation of the examination head 66 by the tilting angle θ (see the arrow XZ2 and the arrow R). The first driving process in the example 2-2 is a process of simultaneously executing the first driving process and the second driving process in the example 2-1 described with reference to Figure 18. With this process, the examination head 66 is moved to the axis TA of tilt, and the optical axis O1 of the objective lens 30 becomes parallel to the axis TA of tilt.

In the first driving process, the examination head 66 may be displaced to a position on the axis TA of tilt where the stereo camera 34 can photograph an anterior eye part of the subject eye E or a position where an observation optical system (not illustrated) inside the examination head 22 can photograph the anterior eye part of the subject eye E via a shortest route.

The drive controlling unit 46 drives the XZ movement mechanism 16 and the Y movement mechanism 62 after completion of the first driving process to execute a second driving process which is the same as the third driving process in the example 2-1, thereby moving the examination head 66 to the examination position along the axis TA of tilt when the examination head 66 is viewed from the one-direction side in the Y direction (see the arrow XZ1). As indicated by reference character XIXC in Figure 19, the drive controlling unit 46 drives the XZ movement mechanism 16 and the Y movement mechanism 62 based on alignment detection performed by the alignment detection unit 44 halfway through the automatic alignment to continue the second driving process until the examination head 66 reaches the examination position.

As described above, in the ophthalmic device 10 according to the second embodiment, the examination head 66 can be moved to the examination position for the subject eye E along the axis TA of tilt tilted at the tilting angle θ at the time of the automatic alignment, like the first embodiment. The same effects as those of the first embodiment can be achieved.

### [Another Method for Determining Tilting Angle θ]

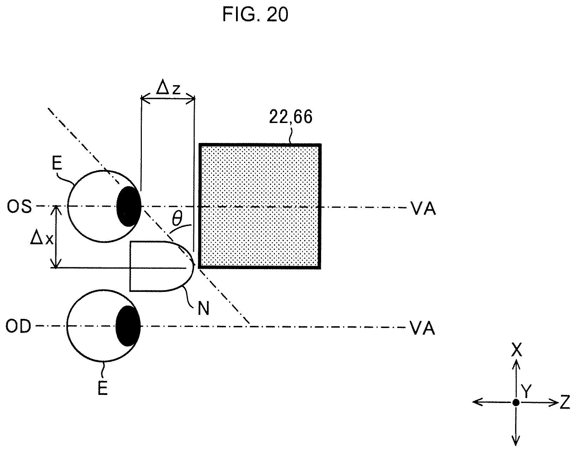

Figure 20 is an explanatory diagram for explaining another method for determining the tilting angle θ by the tilting angle determining unit 43. Although the tilting angle determining unit 43 determines the tilting angle θ based on a result of computing a nose distance Nd for each of a plurality of tilting angles θ different from each other in each of the above-described embodiments, as illustrated in Figure 9, the presently disclosed subject matter is not limited to this.

For example, the tilting angle determining unit 43 may determine the tilting angle θ based on relative position information of a nose N to the examination head 22 and relative position information of the subject eye E to the examination head 22, as illustrated in Figure 20. In this case, the tilting angle determining unit 43 computes the relative position information of the subject eye E to the examination head 22 by detecting an image of the subject eye E from an image of the subject eye E included in a photographed nose image D (see Figure 7) from each camera 34a. The tilting angle determining unit 43 computes the tilting angle θ based on the relative position information of the nose N detected by a nose position detecting unit 42 and the relative position information of the subject eye E.

Specifically, letting ΔZ be an interval in the Z direction between the nose N (tip) and the subject eye E; ΔX, an interval in the X direction between N (tip) and the subject eye E; and A, an arbitrary coefficient, the tilting angle determining unit 43 determines, as the tilting angle θ, an angle expressed by θ = Atan(ΔZ/ΔX).

### [Third Embodiment]

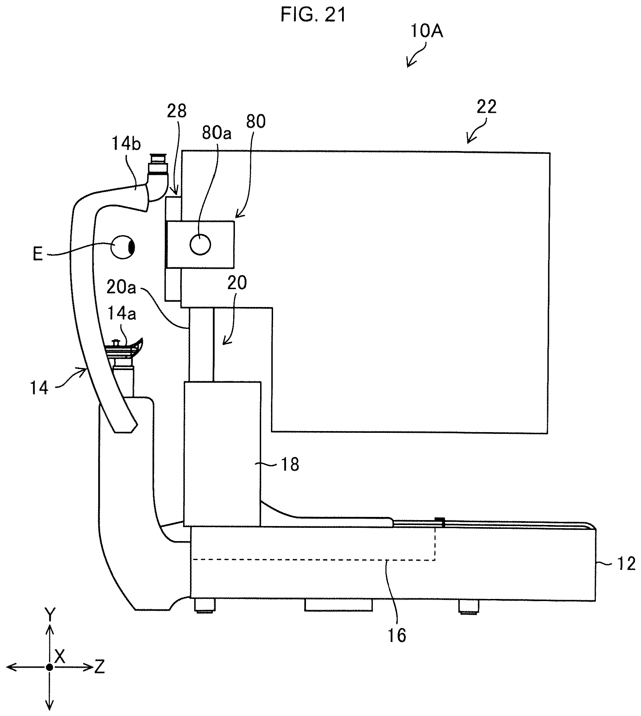

Figure 21 is a side view of an ophthalmic device 10A according to a third embodiment. The ophthalmic device 10 (see Figure 1) according to the above-described first embodiment includes the swing rotation mechanism 20 having the rotating shaft 20a parallel to the Y direction, and brings the examination head 22 closer to the subject eye E along the axis TA of tilt obtained by tilting the reference axis VA in the X direction (the outward direction X1) around the subject eye E at the time of automatic alignment of the examination head 22. In contrast, the ophthalmic device 10A according to the third embodiment brings an examination head 22 closer to a subject eye E along an axis TA of tilt obtained by tilting a reference axis VA in a direction other than an X direction around the subject eye E at the time of automatic alignment of the examination head 22.

As illustrated in Figure 21, the ophthalmic device 10A according to the third embodiment is basically the same in configuration as the ophthalmic device 10 according to the first embodiment except that the ophthalmic device 10A includes a tilt rotation mechanism 80 and executes the automatic alignment of the examination head 22 differently from the first embodiment. For this reason, components functionally or structurally identical to those in the ophthalmic device 10 according to the first embodiment are denoted by identical reference numerals, and a description thereof will be omitted.

The tilt rotation mechanism 80 corresponds to a rotation mechanism according to the presently disclosed subject matter and, together with an XZ movement mechanism 16, a Y movement mechanism 18, and a swing rotation mechanism 20, constitutes a displacement mechanism according to the presently disclosed subject matter. The tilt rotation mechanism 80 includes a rotating shaft 80a parallel to a Y direction and an electric drive mechanism which rotates the rotating shaft 80a, and rotates (tilts) the examination head 22 around the rotating shaft 80a.

A position of the rotating shaft 80a and a position of an objective lens 30 coincide (the term "coincide" as used herein is intended to include the meaning of "coincide substantially"; the same applies hereinafter) when the rotating shaft 80a is viewed from a one-direction side in an axial direction of the rotating shaft 80a. With this configuration, the examination head 22 is rotated (tilted) around the objective lens 30 by the tilt rotation mechanism 80.

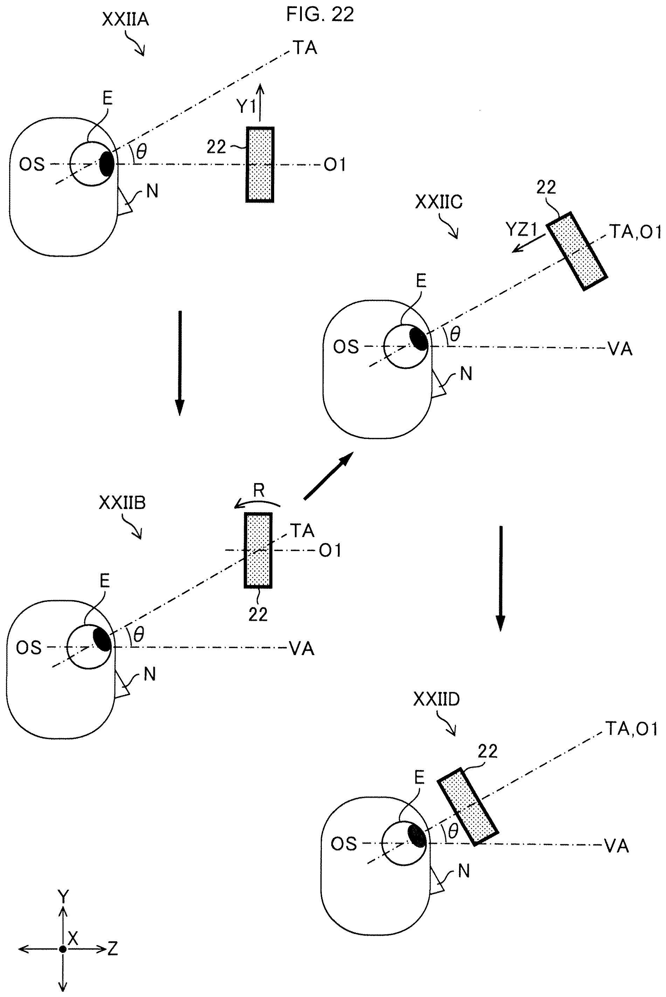

As described above, the examination head 22 according to the third embodiment is biaxially rotatable (swingable and tiltable) around the objective lens 30 by the swing rotation mechanism 20 and the tilt rotation mechanism 80. The examination head 22 according to the third embodiment is thus rotatable around an arbitrary rotating shaft (including ones other than a rotating shaft 20a and the rotating shaft 80a) perpendicular to a Z direction by driving at least one of the swing rotation mechanism 20 and the tilt rotation mechanism 80. For this reason, in the third embodiment, a direction (which is a direction perpendicular to the Z direction and away from a nose N) other than the outward direction X1 according to the first embodiment, such as an upward direction of the Y direction, is set as an outward direction Y1 (see Figure 22), and an axis obtained by tilting the reference axis VA in the outward direction Y1 around the subject eye E is set as the axis TA of tilt.

A control device 40 according to the third embodiment is basically the same as the control device 40 according to the first embodiment except that a direction of tilt of the axis TA of tilt is different from that in the first embodiment.

A tilting angle determining unit 43 according to the third embodiment determines a tilting angle θ in the outward direction Y1 (see Figure 22) of the axis TA of tilt with respect to the reference axis VA when viewed from a one-direction side in the X direction, i.e., the tilting angle θ of the axis TA of tilt with respect to the reference axis VA in a YZ plane based on relative position information of the nose N input from a nose position detecting unit 42. A specific method for determining the tilting angle θ is the same as the method for determining the tilting angle θ according to the first embodiment except that the direction of tilt of the axis TA of tilt is different and that a specific description thereof will be omitted.

A drive controlling unit 46 according to the third embodiment determines the axis TA of tilt based on the tilting angle θ determined by the tilting angle determining unit 43 and then drives the XZ movement mechanism 16, the Y movement mechanism 18, the swing rotation mechanism 20, and the tilt rotation mechanism 80 to execute the automatic alignment of the examination head 22.

Figure 22 is an explanatory diagram for explaining an example 3 of the automatic alignment of the examination head 22 according to the third embodiment. The example 3 is basically the same as the example 1-1 (see Figure 10) described in the first embodiment except that the direction of tilt of the axis TA of tilt is different.

As indicated by reference character XXIIA in Figure 22, the examination head 22 is arranged at the same initial position as the first embodiment at first. The drive controlling unit 46 according to the third embodiment drives the XZ movement mechanism 16 and the Y movement mechanism 18 to execute a first driving process of moving the examination head 22 from the initial position to the axis TA of tilt in the outward direction Y1 (the upward direction of the Y direction) when the examination head 22 is viewed from the one-direction side in the X direction.

As indicated by reference character XXIIB in Figure 22, the drive controlling unit 46 drives the tilt rotation mechanism 80 after completion of the first driving process to execute a second driving process of rotating the examination head 22 around the rotating shaft 80a by the tilting angle θ (see an arrow R). With this process, as indicated by reference character XXIIC in Figure 22, an optical axis O1 of the examination head 22 (the objective lens 30) becomes parallel to the axis TA of tilt. The second driving process may be executed before the first driving process.

The drive controlling unit 46 then drives the XZ movement mechanism 16 and the Y movement mechanism 18 after completion of the second driving process to start a third driving process of moving the examination head 22 to an examination position along the axis TA of tilt when the examination head 22 is viewed from the one-direction side in the X direction (see an arrow YZ1). With this process, the examination head 22 is moved toward the subject eye E while keeping the tilting angle θ constant (the term "constant" as used herein is intended to include the meaning of "substantially constant"; the same applies hereinafter).

As indicated by reference character XXIID in Figure 22, the drive controlling unit 46 drives the XZ movement mechanism 16 and the Y movement mechanism 18 based on alignment detection performed by an alignment detection unit 44 halfway through the automatic alignment to continue the third driving process until the examination head 22 reaches the examination position.

The drive controlling unit 46 switches an alignment mode of the examination head 22 to a manual alignment mode when the alignment detection unit 44 is incapable of alignment detection during a period from the start of the automatic alignment or the start of the third driving process to movement of the examination head 22 for a fixed time determined in advance or over a fixed distance.

Like the example 1-2 (see Figure 11) of the automatic alignment of the examination head 22 according to the first embodiment, the first driving process may be started after the examination head 22 is first moved toward a front side in the Z direction (a subject eye E side) by a predetermined distance. Like the example 1-3 (see Figure 12) of the automatic alignment of the examination head 22 according to the first embodiment, a first driving process of simultaneously executing movement of the examination head 22 toward the front side in the Z direction and in the outward direction Y1 and rotation of the examination head 22 by the tilting angle θ may be executed.

As described above, in the ophthalmic device 10 according to the third embodiment as well, the tilting angle θ (the axis TA of tilt) in the outward direction Y1 can be determined based on photographed nose images D, and the examination head 22 can be moved to the examination position for the subject eye E from an oblique direction (obliquely upward direction) along the axis TA of tilt at the time of the automatic alignment. This prevents the examination head 22 from coming closer to the nose N. As a result, the same effects as those of the first embodiment can be achieved.

Although the examination head 22 is brought closer to the subject eye E along the axis TA of tilt obtained by tilting the reference axis VA in the outward direction Y1 (the upward direction of the Y direction) around the subject eye E at the time of the automatic alignment of the examination head 22 in the third embodiment, the direction of tilt of the axis TA of tilt is not particularly limited as long as the direction is a direction perpendicular to the Z direction and away from the nose N. Directions of the rotating shafts 20a and 80a may be appropriately changed in accordance with the direction of tilt.

If the direction of tilt of the axis TA of tilt is fixed to the outward direction Y1 in the ophthalmic device 10A according to the third embodiment, the swing rotation mechanism 20 may be omitted.

### [Fourth Embodiment]

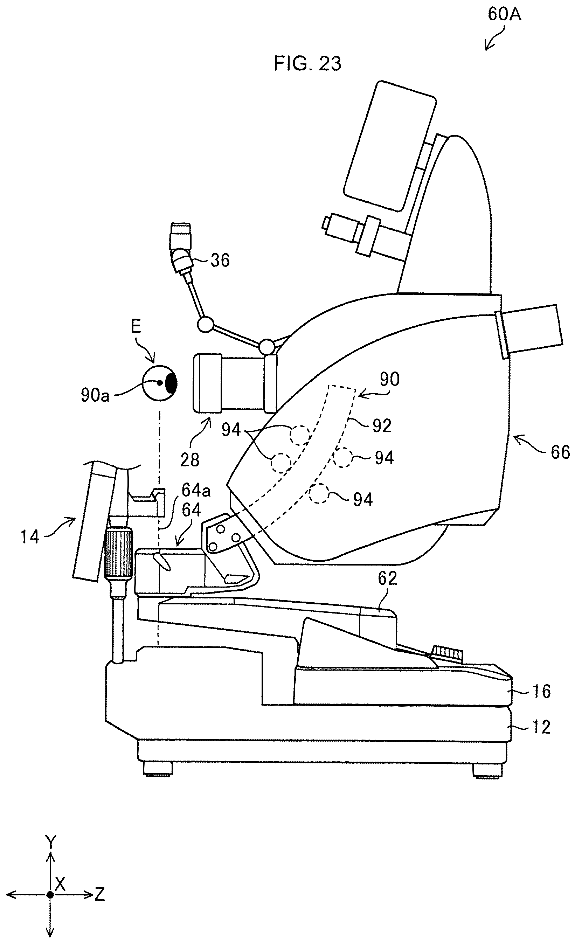

Figure 23 is a side view of an ophthalmic device 60A according to a fourth embodiment. The ophthalmic device 10 (see Figure 17) according to the above-described second embodiment includes the swing rotation mechanism 64 having the rotation axis 64a parallel to the Y direction, and brings the examination head 66 closer to the subject eye E along the axis TA of tilt obtained by tilting the reference axis VA in the X direction (the outward direction X1) around the subject eye E at the time of the automatic alignment of the examination head 66. In contrast, the ophthalmic device 60A according to the fourth embodiment brings an examination head 66 closer to a subject eye E along an axis TA of tilt obtained by tilting a reference axis VA in a direction other than an X direction around the subject eye E at the time of automatic alignment of the examination head 66, like the ophthalmic device 10A according to the above-described third embodiment.

As illustrated in Figure 23, the ophthalmic device 60A according to the fourth embodiment is basically the same in configuration as the ophthalmic device 60 according to the second embodiment except that the ophthalmic device 60A includes a tilt rotation mechanism 90 and executes the automatic alignment of the examination head 66 differently from the second embodiment. For this reason, components functionally or structurally identical to those in the ophthalmic device 60 according to the second embodiment are denoted by identical reference numerals, and a description thereof will be omitted.

The tilt rotation mechanism 90 corresponds to a rotation mechanism according to the presently disclosed subject matter and, together with an XZ movement mechanism 16, a Y movement mechanism 62, and a swing rotation mechanism 64, constitutes a displacement mechanism according to the presently disclosed subject matter. The tilt rotation mechanism 90 rotates (tilts) the examination head 66 around an imaginary rotation axis 90a perpendicular to a Y direction.

The tilt rotation mechanism 90 includes, for example, a curved arm 92, a plurality of guide wheel portions 93, and a head moving mechanism (not illustrated). The curved arm 92 is fixed to the swing rotation mechanism 64 and has the shape of an arc of a circle centered at the rotation axis 90a.

The plurality of guide wheel portions 93 are held inside the examination head 66 so as to be rotatable around center axes parallel to the rotation axis 90a. The guide wheel portions 93 are arranged such that the curved arm 92 is sandwiched therebetween in the Y direction. With this configuration, the examination head 66 is movable along the curved arm 92.

The head moving mechanism (not illustrated) of the tilt rotation mechanism 90 is provided inside the examination head 66 and moves the examination head 66 along the curved arm 92. The configuration of the head moving mechanism is a publicly known technique (e.g., Japanese Patent Application Laid-Open No. 2022-112637) and that a specific description thereof will be omitted. The movement of the examination head 66 along the curved arm 92 by the head moving mechanism allows rotation (tilting) of the examination head 66 around the rotation axis 90a. Additionally, alignment of the rotation axis 90a with the subject eye E allows rotation (tilting) of the examination head 66 around the subject eye E.

As described above, the examination head 66 according to the fourth embodiment is biaxially rotatable (swingable and tiltable) by the swing rotation mechanism 64 and the tilt rotation mechanism 90, like the examination head 22 according to the third embodiment. The examination head 66 according to the fourth embodiment is thus rotatable around an arbitrary rotation axis (including ones other than a rotation axis 64a and the rotation axis 90a) perpendicular to a Z direction by driving at least one of the swing rotation mechanism 64 and the tilt rotation mechanism 90. For this reason, in the fourth embodiment, a direction (which is a direction perpendicular to the Z direction and away from a nose N) other than the outward direction X1 according to the second embodiment, such as an upward direction of the Y direction, is set as an outward direction Y1, and an axis obtained by tilting the reference axis VA in the outward direction Y1 around the subject eye E is set as the axis TA of tilt, like the third embodiment.

A control device 40 according to the fourth embodiment is basically the same as the control device 40 according to the second embodiment except that a direction of tilt of the axis TA of tilt is different from that in the second embodiment.

A tilting angle determining unit 43 according to the fourth embodiment determines a tilting angle θ in the outward direction Y1 of the axis TA of tilt with respect to the reference axis VA based on relative position information of the nose N input from a nose position detecting unit 42, like the tilting angle determining unit 43 according to the third embodiment.

A drive controlling unit 46 according to the fourth embodiment determines the axis TA of tilt based on the tilting angle θ determined by the tilting angle determining unit 43 and then drives the XZ movement mechanism 16, the Y movement mechanism 62, the swing rotation mechanism 64, and the tilt rotation mechanism 90 to execute the automatic alignment of the examination head 66.

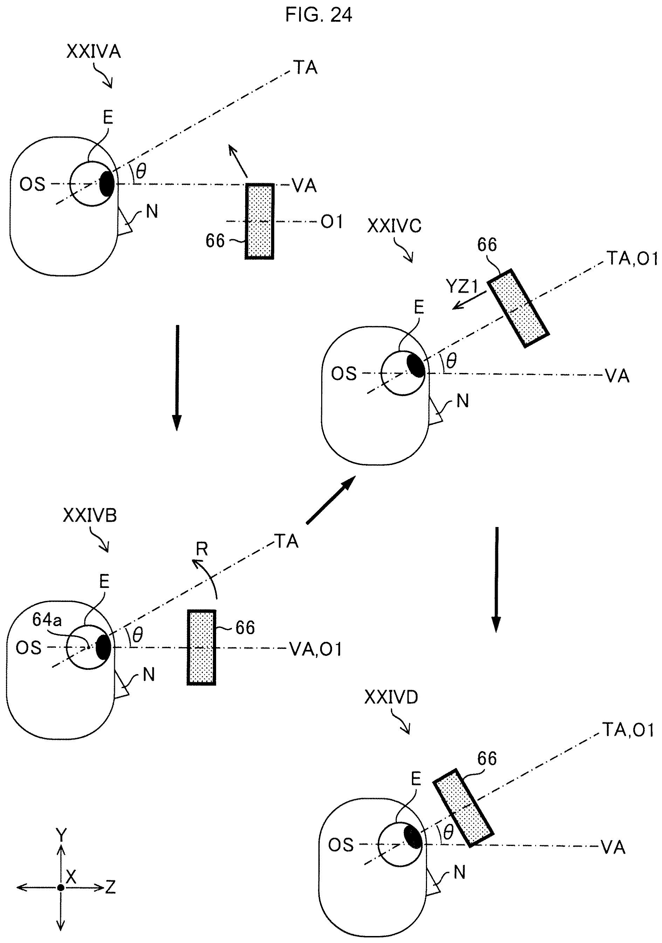

Figure 24 is an explanatory diagram for explaining an example 4 of the automatic alignment of the examination head 66 according to the fourth embodiment. The example 4 is basically the same as the example 2-1 (see Figure 18) described in the second embodiment except that the direction of tilt of the axis TA of tilt is different. As indicated by reference character XXIV A in Figure 24, the examination head 66 is arranged at the same initial position as the first embodiment at first.

As indicated by reference characters XXIVA and XXIVB in Figure 24, the drive controlling unit 46 drives the XZ movement mechanism 16 and the Y movement mechanism 62 to execute a first driving process of moving, in the X, Y, and Z directions, the examination head 66 to a position where the rotation axis 64a and the rotation axis 90a coincide with a circumnutation center of the subject eye E.

The drive controlling unit 46 drives the tilt rotation mechanism 90 after completion of the first driving process to execute a second driving process of rotating the examination head 66 in the outward direction Y1 around the rotation axis 90a (the circumnutation center of the subject eye E) by the tilting angle θ (see an arrow R). With this process, as indicated by reference character XXIVC in Figure 24, the examination head 66 is moved to the axis TA of tilt, and an optical axis O1 becomes parallel to the axis TA of tilt.

The drive controlling unit 46 then drives the XZ movement mechanism 16 and the Y movement mechanism 62 after completion of the second driving process to start a third driving process of moving the examination head 66 to an examination position along the axis TA of tilt when the examination head 66 is viewed from a one-direction side in the X direction (see an arrow YZ1). With this process, the examination head 66 is moved toward the subject eye E while keeping the tilting angle θ constant (the term "constant" as used herein is intended to include the meaning of "substantially constant"; the same applies hereinafter).

As indicated by reference character XXIVD in Figure 24, the drive controlling unit 46 drives the XZ movement mechanism 16 and the Y movement mechanism 62 based on alignment detection performed by an alignment detection unit 44 halfway through the automatic alignment to continue the third driving process until the examination head 66 reaches the examination position.

The drive controlling unit 46 switches an alignment mode of the examination head 66 to a manual alignment mode when the alignment detection unit 44 is incapable of alignment detection during a period from the start of the automatic alignment or the start of the third driving process to movement of the examination head 66 for a fixed time determined in advance or over a fixed distance.

The first driving process and the second driving process may be simultaneously executed, like the example 2-2 (see Figure 19) of the automatic alignment of the examination head 66 according to the second embodiment.

As described above, in the ophthalmic device 10 according to the fourth embodiment as well, the tilting angle θ (the axis TA of tilt) in the outward direction Y1 can be determined based on photographed nose images D, and the examination head 66 can be moved to the examination position for the subject eye E from an oblique direction (obliquely upward direction) along the axis TA of tilt at the time of the automatic alignment. This prevents the examination head 66 from coming closer to the nose N. As a result, the same effects as those of the second embodiment can be achieved.

Although the examination head 66 is brought closer to the subject eye E along the axis TA of tilt obtained by tilting the reference axis VA in the outward direction Y1 (the upward direction of the Y direction) around the subject eye E at the time of the automatic alignment of the examination head 66 in the fourth embodiment, the direction of tilt of the axis TA of tilt around the subject eye E is not particularly limited as long as the direction is a direction perpendicular to the Z direction and away from the nose N. Directions of the rotation axes 64a and 90a may be appropriately changed in accordance with the direction of tilt.

When the direction of tilt of the axis TA of tilt is fixed to the outward direction Y1 in the ophthalmic device 10A according to the fourth embodiment, the swing rotation mechanism 64 may be omitted.

### [Others]

Although alignment detection is executed using the stereo camera 34 in each of the above-described embodiments, the alignment detection may be executed using an observation optical system (not illustrated) housed in the examination head 22 or 66. In this case, the stereo camera 34 may be omitted. Although a position of the nose N of the subject is detected using the stereo camera 34 in each embodiment, the face (nose N) of the subject is photographed using the above-described various photographing units including an observation optical system, and the position of the nose N may be detected based on photographed images obtained through the photographing.

Although the stereo camera 34 is provided at the lens-barrel distal end face 28a in each embodiment, the stereo camera 34 may be provided at a position other than ones at the lens-barrel distal end face 28a in the examination head 22 or 66.

Although the nose N is photographed by all the cameras 34a constituting the stereo camera 34 in each embodiment, photographing of the nose N by two cameras 34a allows position detection of the nose N by the nose position detecting unit 42. For this reason, when the total number of cameras 34a is three or more, the nose N need not be photographed by all the cameras 34a, and the nose N may be photographed by at least two cameras 34a.

Although the examination head 22 or 66 is moved from an oblique direction to the examination position for the subject eye E along the axis TA of tilt with the tilting angle θ determined by the tilting angle determining unit 43 at the time of automatic alignment in each embodiment, the examination head 22 or 66 may be moved from an oblique direction to the examination position for the subject eye E along the axis TA of tilt with the tilting angle θ at the time of manual alignment.

Although a case where a displacement mechanism which displaces the examination head 22 or 66 with respect to the subject eye E includes the XZ movement mechanism 16, the Y movement mechanism 18 or 62, and the swing rotation mechanism 20 or 64 and a case where the displacement mechanism includes the tilt rotation mechanism 80 or 90 in addition to the listed components have been described as examples in the embodiments, a configuration and the type of the displacement mechanism are not particularly limited. For example, a robot arm (multijoint arm) may be used as a displacement mechanism according to the presently disclosed subject matter.

Although a multifunction machine which is a combination of a fundus camera and an optical coherence tomograph and a fundus camera alone have been described as examples of the ophthalmic device 10 in the embodiments, the presently disclosed subject matter is not limited to this. The presently disclosed subject matter can also be applied to various ophthalmic devices (including devices which perform various procedures on the subject eye E, such as a laser surgery device) which are used for examination (ocular characteristics measurement, photographing, and observation) on the subject eye E and execute alignment of various examination heads with the subject eye E, such as an optical coherence tomograph alone and an SLO device.

### {Reference Signs List}

10 ophthalmic device
12 base
14 face support
14a chin rest
14b forehead rest
16 XZ movement mechanism
18 Y movement mechanism
20 swing rotation mechanism
20a rotating shaft
22 examination head
24 fundus camera unit
26 OCT unit
28 lens-barrel
28a lens-barrel distal end face
30 objective lens
32 illumination light source
34 stereo camera
34a camera
36 vision fixation light emitting unit
37 display unit
38 manipulation unit
39 storage unit
40 control device
41 nose photographing controlling unit
42 nose position detecting unit
43 tilting angle determining unit
44 alignment detection unit
46 drive controlling unit
48 vision fixation controlling unit
50 measurement controlling unit
52 saving controlling unit
60 ophthalmic device
62 Y movement mechanism
64 swing rotation mechanism
64a rotation axis
66 examination head
80, 90 tilt rotation mechanism
80a, 90a rotating shaft, rotation axis
100 objective lens
102 objective lens
104 examination head
D photographed nose image
E subject eye
F1 upper region
F2 lower oblique region
H subject
N nose
Nd nose distance
O1 optical axis
O2 photographing optical axis
OD right eye
OS left eye
TA axis of tilt
VA reference axis
X1 outward direction
Y1 outward direction
d operating distance
d1 operating distance
d2 operating distance
α angle
θ tilting angle

## Claims

1. An ophthalmic device comprising:
an examination head configured to examine a subject eye;
a displacement mechanism configured to displace the examination head with respect to the subject eye;
a plurality of cameras provided at the examination head;
a nose photographing controlling unit configured to cause at least two of the cameras to photograph a nose of a subject from a plurality of directions different from each other;
a nose position detecting unit configured to detect a relative position of the nose to the examination head based on respective photographed images of the nose photographed by the cameras;
a tilting angle determining unit configured to, when an axis along an eye direction of the subject eye parallel to a front-back direction which is an operating distance direction of the examination head is a reference axis, and an axis obtained by tilting the reference axis in an outward direction away from the nose of the subject around the subject eye is an axis of tilt, determine a tilting angle of the axis of tilt with respect to the reference axis based on a result of detection by the nose position detecting unit; and
a drive controlling unit configured to drive the displacement mechanism to displace the examination head to an examination position for the subject eye along the axis of tilt with the tilting angle determined by the tilting angle determining unit.

2. The ophthalmic device according to claim 1, wherein the nose photographing controlling unit causes all of the cameras to simultaneously photograph the nose after positionally adjusting, by the displacement mechanism, the examination head to a position where the nose is photographable by all the cameras.

3. The ophthalmic device according to claim 1, wherein
the nose photographing controlling unit executes
a divisional photographing process of causing one or more of the cameras to photograph the nose after driving the displacement mechanism to move the examination head to a position where the nose is photographable by the one or more cameras,
a position information acquiring process of acquiring photographing position information of each of the cameras that have photographed the nose in the divisional photographing process, and
a repetition process of repeatedly executing the divisional photographing process and the position information acquiring process until photographing of the nose by all the cameras is completed, and
the nose position detecting unit detects the relative position of the nose based on the respective photographed images from and the respective pieces of photographing position information of the cameras.

4. The ophthalmic device according to claim 1, wherein the tilting angle determining unit computes, for each of a plurality of tilting angles different from each other and constituting the tilting angle, a nose distance which is a distance between the examination head and the nose when the ophthalmic device brings the examination head closer to an operating distance determined in advance from the subject eye based on a result of detection by the nose position detecting unit and determines the tilting angle based on a result of computation of the respective nose distances for the tilting angles.

5. The ophthalmic device according to claim 1, wherein
the plurality of cameras photograph an anterior eye part of the subject eye halfway through displacement of the examination head to the examination position by the displacement mechanism,
the ophthalmic device comprises an alignment detection unit configured to detect a relative position of the subject eye to the examination head based on anterior eye part images of the subject eye photographed by the plurality of cameras, and
the drive controlling unit drives the displacement mechanism based on a result of detection by the alignment detection unit to execute alignment of the examination head with the subject eye while maintaining the tilting angle determined by the tilting angle determining unit.

6. The ophthalmic device according to any one of claims 1 to 5, wherein
the examination head includes an objective lens and a lens-barrel configured to hold the objective lens, and
one of the plurality of cameras is provided at a position below the objective lens in a distal end face of the lens-barrel.

7. The ophthalmic device according to claim 6, wherein
the cameras are provided at the position below the objective lens and positions on the left and right of the objective lens in the distal end face of the lens-barrel.

8. The ophthalmic device according to any one of claims 1 to 5, wherein
the displacement mechanism includes
a movement mechanism configured to move the examination head with respect to the subject eye in the front-back direction, a left-right direction, and an up-down direction, and
a rotation mechanism configured to rotate the examination head around a rotation axis determined in advance.

9. The ophthalmic device according to claim 8, wherein
the rotation axis is parallel to the up-down direction, and
the outward direction is parallel to the left-right direction.

10. The ophthalmic device according to claim 8, wherein
the rotation axis is perpendicular to the up-down direction, and
the outward direction is an upward direction of the up-down direction.

11. A method for operating an ophthalmic device, the ophthalmic device including
an examination head configured to examine a subject eye,
a displacement mechanism configured to displace the examination head with respect to the subject eye, and
a plurality of cameras provided to be movable integrally with the examination head, the method comprising:
a nose photographing controlling step of causing at least two of the cameras to photograph a nose of a subject from a plurality of directions different from each other;
a nose position detecting step of detecting a relative position of the nose to the examination head based on respective photographed images of the nose photographed by the cameras;
a tilting angle determining step of, when an axis along an eye direction of the subject eye parallel to a front-back direction which is an operating distance direction of the examination head is a reference axis, and an axis obtained by tilting the reference axis in an outward direction away from the nose of the subject around the subject eye is an axis of tilt, determining a tilting angle of the axis of tilt with respect to the reference axis based on a result of detection in the nose position detecting step; and
a drive controlling step of driving the displacement mechanism to displace the examination head to an examination position for the subject eye along the axis of tilt with the tilting angle determined in the tilting angle determining step.
